# EUROPEAN PATENT SPECIFICATION

(11) **EP 3 587 597 B1**
(45) Date of publication and mention of the grant of the patent: **11.10.2023**
(21) Application number: 18758093.1
(22) Date of filing: 23.02.2018
(51) Int. Cl.: C12Q 1/6883, C12Q 1/689

(54) **METHOD FOR DIAGNOSING PARKINSON'S DISEASE THROUGH BACTERIAL METAGENOME ANALYSIS**
VERFAHREN ZUR DIAGNOSE VON MORBUS PARKINSON DURCH BAKTERIELLE METAGENOMANALYSE
PROCÉDÉ DE DIAGNOSTIC DE LA MALADIE DE PARKINSON PAR L'ANALYSE DU MÉTAGÉNOME BACTÉRIEN

(30) Priority: 24.02.2017 KR 20170025001; 22.02.2018 KR 20180021196
(43) Date of publication of application: 01.01.2020
(73) Proprietor: MD Healthcare Inc., Seoul 03923 (KR)
(72) Inventor: KIM, Yoon-Keun, Namyangju-si Gyeonggi-do 12146 (KR)
(74) Representative: Dehns
(86) International application number: PCT/KR2018/002281
(87) International publication number: WO 2018/155961

(56) References cited:
- WO-A1-2011/127219
- WO-A1-2015/181449
- WO-A1-2017/009693
- WO-A1-2019/004668
- WO-A1-2019/156449
- WO-A1-2019/164197
- WO-A1-2019/164230
- KR-A- 20110 025 603
- KR-A- 20110 138 124
- KR-A- 20160 073 157
- MADELYN C. HOUSER ET AL: "The gut-brain axis: is intestinal inflammation a silent driver of Parkinson's disease pathogenesis?", NPJ PARKINSON'S DISEASE, vol. 3, no. 1, 11 January 2017 (2017-01-11), XP055743701, DOI: 10.1038/s41531-016-0002-0
- Maurizio Muraca: "Gut Microbiota-derived Outer Membrane Vesicles: Under-recognized Major Players in Health and Disease?", , 26 May 2015 (2015-05-26), XP055743820, Retrieved from the Internet: URL:https://www.discoverymedicine.com/Maur izio-Muraca/2015/05/gut-microbiota-derived -outer-membrane-vesicles-under-recognized- major-players-in-health-and-disease/ [retrieved on 2020-10-26]
- ALI KESHAVARZIAN ET AL: "Colonic bacterial composition in Parkinson's disease : COLONIC MICROBIOTA IN PARKINSON'S DISEASE", MOVEMENT DISORDERS, vol. 30, no. 10, 16 July 2015 (2015-07-16) , pages 1351-1360, XP055494681, US ISSN: 0885-3185, DOI: 10.1002/mds.26307
- ERIN M. HILL-BURNS ET AL: "Parkinson's disease and Parkinson's disease medications have distinct signatures of the gut microbiome : PD, Medications, and Gut Microbiome", MOVEMENT DISORDERS, vol. 32, no. 5, 14 February 2017 (2017-02-14), pages 739-749, XP055743698, US ISSN: 0885-3185, DOI: 10.1002/mds.26942
- JAE YOUNG YOO ET AL: "16S rRNA gene-based metagenomic analysis reveals differences in bacteria-derived extracellular vesicles in the urine of pregnant and non-pregnant women", EXPERIMENTAL & MOLECULAR MEDICINE, vol. 48, no. 2, 5 February 2016 (2016-02-05), page e208, XP055321074, DOI: 10.1038/emm.2015.110

## Description

### [Technical Field]

The present invention relates to a method of diagnosing Parkinson's disease by analyzing an increase or decrease in content of extracellular vesicles derived from specific bacteria by bacterial metagenomic analysis using a subject-derived urine sample.

### [Background Art]

Parkinson's disease is a progressive neurodegenerative disease characterized by Parkinsonian symptoms such as slow motion, tremors at rest, muscle stiffness, abnormal gait characteristics, and a bending posture. The disease occurs due to a decrease in stimulation of the motor cortex which results from imperfect dopamine production and action, mainly in the substantia nigra. Serious cognitive impairment and mild language impairment also occur and they are chronic and progressive. Parkinson's disease may also occur even when contracting Japanese encephalitis, brain syphilis, carbon dioxide poisoning, manganese poisoning, or Wilson's disease. The probability of developing the disease is one in 1,000, but incidence increases with age. In addition, movement disorders occur, making it uncomfortable to move.

Unlike most other serious neurological or psychological disorders, Parkinson's disease has a relatively low hereditary nature. Most patients do not have relatives with Parkinson's disease. In a case in which one of the identical twins contracts the disease, the probability for the other person to contract the disease is not even 10%. This is, of course, clearly higher than a 0.1% probability that other people contract the disease, but is not so high. Incidence-related environmental factors include blockage of blood flowing to specific areas of the brain, long-term exposure to certain drugs and poisons, and history of being infected by encephalitis or other viruses.

Meanwhile, it is known that the number of microorganisms symbiotically living in the human body is 100 trillion which is 10 times the number of human cells, and the number of genes of microorganisms exceeds 100 times the number of human genes. A microbiota is a microbial community that includes bacteria, archaea, and eukaryotes present in a given habitat. The intestinal microbiota is known to play a vital role in human's physiological phenomena and significantly affect human health and diseases through interactions with human cells. Bacteria coexisting in human bodies secrete nanometer-sized vesicles to exchange information about genes, proteins, and the like with other cells. The mucous membranes form a physical barrier membrane that does not allow particles with the size of 200 nm or more to pass therethrough, and thus bacteria symbiotically living in the mucous membranes are unable to pass therethrough, but bacteria-derived extracellular vesicles have a size of approximately 100 nm or less and thus relatively freely pass through the mucous membranes and are absorbed into the human body.

Metagenomics, also called environmental genomics, may be analytics for metagenomic data obtained from samples collected from the environment (Korean Patent Publication No. 2011-0073049). Recently, the bacterial composition of human microbiota has been listed using a method based on 16s ribosomal RNA (16s rRNA) base sequences, and 16s rDNA base sequences, which are genes of 16s ribosomal RNA, are analyzed using a next generation sequencing (NGS) platform. However, in the onset of Parkinson's disease, identification of causative factors of Parkinson's disease through metagenomic analysis of bacteria-derived vesicles isolated from a human-derived substance, such as urine and the like, and a method of diagnosing Parkinson's disease have never been reported.

Houser et al., (2017) NPJ Parkinson's Disease 3(1) DOI:10.1038/s41531-016-0002-0 discloses a model for PD pathogenesis in which the disorder originates in the intestine and progresses with inflammation as its underlying mechanism, and that fecal microbiota from PD patients appears deficient in anti-inflammatory microbes.

Muraca et al., (2015) "Gut Microbiota-derived Outer Membrane Vesicles: Under-recognized Major Players in Health and Disease?" discloses that gut microbiota-derived outer membrane vesicles contain DNA and can enter systemic circulation.

Keshavarzia et al., (2015) Movement Disorders 30(10): 1351-1360 discloses altered gut microbiota present in PD patients.

Hill-Burns et al, (2017) Movement Disorders 32(5): 739-749 discloses altered gut microbiota present in PD patients.

To pre-diagnose causative factors of Parkinson's disease and a risk of developing the disease, the inventors of the present invention extracted DNA from bacteria-derived extracellular vesicles present in urine, which is a subject-derived sample, and performed metagenomic analysis on the extracted DNA, and, as a result, identified bacteria-derived extracellular vesicles capable of acting as a causative factor of Parkinson's disease, and thus completed the present invention based on these findings.

The present invention provides a method of diagnosing Parkinson's disease as defined in the appended set of claims

In the present invention, the subject sample is urine.

In the present invention, process (c) comprises comparing an increase or decrease in content of bacteria-derived extracellular vesicles of the subject sample with that of a normal individual-derived sample through sequencing of a product of the PCR, wherein said extracellular vesicles are derived from:
one or more bacteria selected from the group consisting of the phylum *Proteobacteria,* the phylum *Cyanobacteria,* the phylum *Gemmatimonadetes,* the phylum *Chloroflexi,* the phylum *Synergistetes,* the phylum *Acidobacteria,* the phylum *Planctomycetes,* the phylum *Parvarchaeota,* the phylum *Chlorobi,* and the phylum *Thermotogae;*
one or more bacteria selected from the group consisting of the class *Coriobacteriia,* the class *Gammaproteobacteria,* the class *Verrucomicrobiae,* the class *Actinobacteria,* the class *Alphaproteobacteria,* the class *Chloroplast,* the class *Saprospirae,* the class *Deltaproteobacteria,* the class *Epsilonproteobacteria,* the class Ellin6529, the class *Chloracidobacteria,* the class *Opitutae,* the class *Thermoleophilia,* the class *Synergistia,* the class *Gemmatimonadetes,* the class *Planctomycetia,* the class *Acidobacteriia,* the class *Solibacteres,* the class *Anaerolineae,* the class *Chloroflexi,* the class *Phycisphaerae,* the class *Synechococcophycideae,* the class *Acidimicrobiia,* the class *Acidobacteria-*6*,* the class *Spartobacteria,* the class *Pedosphaerae,* the class *Ktedonobacteria,* and the class *Dehalococcoidetes*;
one or more bacteria selected from the group consisting of the order *Turicibacterales,* the order *Pseudomonadales,* the order *Coriobacteriales,* the order *Pasteurellales,* the order *Enterobacteriales,* the order *Verrucomicrobiales,* the order *Gemellales,* the order *Neisseriales,* the order *Saprospirales,* the order *Actinomycetales,* the order *Streptophyta,* the order *Rhizobiales,* the order *Rhodospirillales,* the order *Xanthomonadales,* the order *Myxococcales,* the order *Campylobacterales,* the order *Desulfovibrionales,* the order *Solirubrobacterales,* the order *Opitutales,* the order *Rickettsiales,* the order *Pirellulales,* the order *Synergistales,* the order *Planctomycetales,* the order *Acidobacteriales,* the order *Solibacterales,* the order *Gaiellales,* the order *Gemmatales,* the order *Acidimicrobiales,* the order *Chthoniobacterales,* the order *Thermoanaerobacterales,* the order *Pedosphaerales,* the order *Phycisphaerales,* the order Sediment-1, the order *Chlorophyta,* the order *Synechococcales,* the order *Roseiflexales,* the order Ellin329, the order *Anaerolineales,* the order Ellin5290, the order *Cryptophyta,* and the order *Thermotogales;*
one or more bacteria selected from the group consisting of the family *Exiguobacteraceae,* the family *Enterococcaceae,* the family *Turicibacteraceae,* the family *Mogibacteriaceae,* the family *Moraxellaceae,* the family *Porphyromonadaceae,* the family *Burkholderiaceae,* the family *Actinomycetaceae,* the family *Coriobacteriaceae,* the family *Methylobacteriaceae,* the family *Streptococcaceae,* the family *Pseudomonadaceae,* the family *Pasteurellaceae,* the family *Veillonellaceae,* the family *Peptostreptococcaceae,* the family *Enterobacteriaceae,* the family *Verrucomicrobiaceae,* the family *Lachnospiraceae,* the family *Bradyrhizobiaceae,* the family *Rikenellaceae,* the family *Tissierellaceae,* the family *Bacteroidaceae,* the family *Chitinophagaceae,* the family *Corynebacteriaceae,* the family *Xanthomonadaceae,* the family *Rhizobiaceae,* the family *Propionibacteriaceae,* the family *Desulfobacteraceae,* the family *Barnesiellaceae,* the family *Comamonadaceae,* the family *mitochondria,* the family *Hyphomicrobiaceae,* the family *Alteromonadaceae,* the family *Sinobacteraceae,* the family *Pirellulaceae,* the family *Dethiosulfovibrionaceae,* the family *Acidobacteriaceae,* the family *Planctomycetaceae,* the family *Isosphaeraceae,* the family *Gaiellaceae,* the family *Koribacteraceae,* the family *Helicobacteraceae,* the family *Chthoniobacteraceae,* the family *Gemmataceae,* the family *Solibacteraceae,* the family *Pelagibacteraceae,* the family Ellin515, the family *Thermoanaerobacteraceae,* the family *Methanoregulaceae,* the family *Synechococcaceae,* the family *Desulfomicrobiaceae,* the family *Kouleothrixaceae,* the family *Alicyclobacillaceae,* the family *Myxococcaceae,* the family *Anaerolinaceae,* and the family *Desulfohalobiaceae*;
one or more bacteria selected from the group consisting of the genus *Collinsella,* the genus *Adlercreutzia,* the genus *Exiguobacterium,* the genus *Enterococcus,* the genus *Acinetobacter,* the genus *Turicibacter,* the genus *Klebsiella,* the genus *Lautropia,* the genus *Akkermansia,* the genus *Parabacteroides,* the genus *Rhizobium,* the genus *Actinomyces,* the genus *Veillonella,* the genus *Pseudomonas,* the genus *Rothia,* the genus *Dorea,* the genus *Streptococcus,* the genus *Haemophilus,* the genus *Enhydrobacter,* the genus *Rhodococcus,* the genus *Coprococcus,* the genus *Oscillospira,* the genus *Ruminococcus,* the genus *Bacteroides,* the genus *Corynebacterium,* the genus *Weissella,* the genus *Propionibacterium,* the genus *Lysinibacillus,* the genus *Stenotrophomonas,* the genus *Arthrobacter,* the genus *Comamonas,* the genus *Marinobacter,* the genus *Clostridium,* the genus *Planctomyces,* the genus *Luteolibacter,* the genus *Delftia,* the genus *Agrobacterium,* the genus *Rhodoplanes,* the genus *Gemmata,* the genus *Coprobacillus,* the genus *Arcobacter,* the genus *Helicobacter,* the genus *Candidatus Solibacter,* the genus *Methanosarcina,* the genus *Thermacetogenium,* the genus *Synechococcus,* the genus *Desulfomicrobium,* the genus *Chthoniobacter,* the genus *Aminobacterium,* the genus *Gallicola,* the genus *Anaeromyxobacter,* the genus *Muricauda,* and the genus *Candidatus Koribacter,*
wherein the subject sample is urine.

### [Advantageous Effects]

Extracellular vesicles secreted from microorganisms such as bacteria, archaea, and the like present in the environment are absorbed into the human body, and thus can directly affect the occurrence of inflammation, and since it is difficult to implement early diagnosis for Parkinson's disease, which is characterized by inflammatory responses, before symptoms appear, efficient treatment therefor is difficult. Thus, according to the present invention, Parkinson's disease may be diagnosed through metagenomic analysis of bacteria-derived extracellular vesicles using a human body-derived urine sample, and thus the onset of Parkinson's disease can be delayed or prevented through appropriate management by early diagnosis, and, even after Parkinson's disease occurs, early diagnosis for Parkinson's disease can be implemented, thereby lowering the incidence rate of Parkinson's disease and increasing therapeutic effects. In addition, patients diagnosed with Parkinson's disease are able to avoid exposure to causative factors predicted through metagenomic analysis, whereby the progression of Parkinson's disease is ameliorated, or recurrence thereof can be prevented.

### [Description of Drawings]

FIG. 1A illustrates images showing the distribution pattern of bacteria and extracellular vesicles over time after intestinal bacteria and bacteria-derived extracellular vesicles (EVs) were orally administered to mice, and FIG. 1B illustrates images showing the distribution pattern of bacteria and EVs after being orally administered to mice and, at 12 hours, blood and various organs were extracted.
FIG. 2 illustrates distribution results of bacteria-derived EVs exhibiting significant diagnostic performance at a phylum level, after metagenomic analysis of bacteria-derived EVs isolated from Parkinson's disease patient-derived urine and normal individual-derived urine.
FIG. 3 illustrates distribution results of bacteria-derived EVs exhibiting significant diagnostic performance at a class level, after metagenomic analysis of bacteria-derived EVs isolated from Parkinson's disease patient-derived urine and normal individual-derived urine.
FIG. 4 illustrates distribution results of bacteria-derived EVs exhibiting significant diagnostic performance at an order level, after metagenomic analysis of bacteria-derived EVs isolated from Parkinson's disease patient-derived urine and normal individual-derived urine.
FIG. 5 illustrates distribution results of bacteria-derived EVs exhibiting significant diagnostic performance at a family level, after metagenomic analysis of bacteria-derived EVs isolated from Parkinson's disease patient-derived urine and normal individual-derived urine.
FIG. 6 illustrates distribution results of bacteria-derived EVs exhibiting significant diagnostic performance at a genus level, after metagenomic analysis of bacteria-derived EVs isolated from Parkinson's disease patient-derived urine and normal individual-derived urine.

### [Best Mode]

The present invention relates to a method of diagnosing Parkinson's disease through bacterial metagenomic analysis as defined in the appended set of claims. The inventors of the present invention extracted genes from bacteria-derived extracellular vesicles using a subject-derived urine sample, performed metagenomic analysis thereon, and identified bacteria-derived extracellular vesicles capable of acting as a causative factor of Parkinson's disease.

The term "Parkinson's disease diagnosis" as used herein refers to determining whether a patient has a risk for Parkinson's disease, whether the risk for Parkinson's disease is relatively high, or whether Parkinson's disease has already occurred. The method of the present invention may be used to delay the onset of Parkinson's disease through special and appropriate care for a specific patient, which is a patient having a high risk for Parkinson's disease or prevent the onset of Parkinson's disease. In addition, the method may be clinically used to determine treatment by selecting the most appropriate treatment method through early diagnosis of Parkinson's disease.

The term "metagenome" as used herein refers to the total of genomes including all viruses, bacteria, fungi, and the like in isolated regions such as soil, the intestines of animals, and the like, and is mainly used as a concept of genomes that explains identification of many microorganisms at once using a sequencer to analyze non-cultured microorganisms. In particular, a metagenome does not refer to a genome of one species, but refers to a mixture of genomes, including genomes of all species of an environmental unit. This term originates from the view that, when defining one species in a process in which biology is advanced into omics, various species as well as existing one species functionally interact with each other to form a complete species. Technically, it is the subject of techniques that analyzes all DNAs and RNAs regardless of species using rapid sequencing to identify all species in one environment and verify interactions and metabolism. In the present invention, bacterial metagenomic analysis is performed using bacteria-derived extracellular vesicles isolated from, for example, serum.

The term "bacteria-derived vesicles" as used herein is a concept comprising extracellular vesicles derived from bacteria.

In the present invention, the subject sample is urine.

In the present invention, metagenomic analysis is performed on the bacteria-derived extracellular vesicles, and bacteria-derived extracellular vesicles capable of acting as a cause of the onset of Parkinson's disease were actually identified by analysis at phylum, class, order, family, and genus levels.

More particularly, in one embodiment of the present invention, as a result of performing bacterial metagenomic analysis on extracellular vesicles present in subject-derived urine samples at a phylum level, the content of extracellular vesicles derived from bacteria belonging to the phylum *Proteobacteria,* the phylum *Cyanobacteria,* the phylum *Gemmatimonadetes,* the phylum *Chloroflexi,* the phylum *Synergistetes,* the phylum *Acidobacteria,* the phylum *Planctomycetes,* the phylum *Parvarchaeota,* the phylum *Chlorobi,* and the phylum *Thermotogae* was significantly different between Parkinson's disease patients and normal individuals (see Example 4).

More particularly, in one embodiment of the present invention, as a result of performing bacterial metagenomic analysis on extracellular vesicles present in subject-derived urine samples at a class level, the content of extracellular vesicles derived from bacteria belonging to the class *Coriobacteriia,* the class *Gammaproteobacteria,* the class *Verrucomicrobiae,* the class *Actinobacteria,* the class *Alphaproteobacteria,* the class *Chloroplast,* the class *Saprospirae,* the class *Deltaproteobacteria,* the class *Epsilonproteobacteria,* the class Ellin6529, the class *Chloracidobacteria,* the class *Opitutae,* the class *Thermoleophilia,* the class *Synergistia,* the class *Gemmatimonadetes,* the class *Planctomycetia,* the class *Acidobacteriia,* the class *Solibacteres,* the class *Anaerolineae,* the class *Chloroflexi,* the class *Phycisphaerae,* the class *Synechococcophycideae,* the class *Acidimicrobiia,* the class *Acidobacteria-*6*,* the class *Spartobacteria,* the class *Pedosphaerae,* the class *Ktedonobacteria,* and the class *Dehalococcoidetes,* was significantly different between Parkinson's disease patients and normal individuals (see Example 4).

More particularly, in one embodiment of the present invention, as a result of performing bacterial metagenomic analysis on extracellular vesicles present in subject-derived urine samples at an order level, the content of extracellular vesicles derived from bacteria belonging to the order *Turicibacterales,* the order *Pseudomonadales,* the order *Coriobacteriales,* the order *Pasteurellales,* the order *Enterobacteriales,* the order *Verrucomicrobiales,* the order *Gemellales,* the order *Neisseriales,* the order *Saprospirales,* the order *Actinomycetales,* the order *Streptophyta,* the order *Rhizobiales,* the order *Rhodospirillales,* the order *Xanthomonadales,* the order *Myxococcales,* the order *Campylobacterales,* the order *Desulfovibrionales,* the order *Solirubrobacterales,* the order *Opitutales,* the order *Rickettsiales,* the order *Pirellulales,* the order *Synergistales,* the order *Planctomycetales,* the order *Acidobacteriales,* the order *Solibacterales,* the order *Gaiellales,* the order *Gemmatales,* the order *Acidimicrobiales,* the order *Chthoniobacterales,* the order *Thermoanaerobacterales,* the order *Pedosphaerales,* the order *Phycisphaerales,* the order Sediment-1, the order *Chlorophyta,* the order *Synechococcales,* the order *Roseiflexales,* the order Ellin329, the order *Anaerolineales,* the order Ellin5290, the order *Cryptophyta,* and the order *Thermotogales* was significantly different between Parkinson's disease patients and normal individuals (see Example 4).

More particularly, in one embodiment of the present invention, as a result of performing bacterial metagenomic analysis on extracellular vesicles present in subject-derived urine samples at a family level, the content of extracellular vesicles derived from bacteria belonging to the family *Exiguobacteraceae,* the family *Enterococcaceae*, the family *Turicibacteraceae*, the family *Mogibacteriaceae*, the family *Moraxellaceae*, the family *Porphyromonadaceae*, the family *Burkholderiaceae*, the family *Actinomycetaceae*, the family *Coriobacteriaceae*, the family *Methylobacteriaceae*, the family *Streptococcaceae*, the family *Pseudomonadaceae*, the family *Pasteurellaceae*, the family *Veillonellaceae*, the family *Peptostreptococcaceae*, the family *Enterobacteriaceae*, the family *Verrucomicrobiaceae*, the family *Lachnospiraceae*, the family *Bradyrhizobiaceae*, the family *Rikenellaceae*, the family *Tissierellaceae*, the family *Bacteroidaceae*, the family *Chitinophagaceae*, the family *Corynebacteriaceae*, the family *Xanthomonadaceae*, the family *Rhizobiaceae*, the family *Propionibacteriaceae*, the family *Desulfobacteraceae*, the family *Barnesiellaceae*, the family *Comamonadaceae*, the family *mitochondria*, the family *Hyphomicrobiaceae*, the family *Alteromonadaceae*, the family *Sinobacteraceae*, the family *Pirellulaceae*, the family *Dethiosulfovibrionaceae*, the family *Acidobacteriaceae*, the family *Planctomycetaceae*, the family *Isosphaeraceae*, the family *Gaiellaceae*, the family *Koribacteraceae*, the family *Helicobacteraceae*, the family *Chthoniobacteraceae*, the family *Gemmataceae*, the family *Solibacteraceae*, the family *Pelagibacteraceae*, the family Ellin515, the family *Thermoanaerobacteraceae*, the family *Methanoregulaceae*, the family *Synechococcaceae*, the family *Desulfomicrobiaceae*, the family *Kouleothrixaceae*, the family *Alicyclobacillaceae*, the family *Myxococcaceae*, the family *Anaerolinaceae*, and the family *Desulfohalobiaceae* was significantly different between Parkinson's disease patients and normal individuals (see Example 4).

More particularly, in one embodiment of the present invention, as a result of performing bacterial metagenomic analysis on extracellular vesicles present in subject-derived urine samples at a genus level, the content of extracellular vesicles derived from bacteria belonging to the genus *Collinsella*, the genus *Adlercreutzia*, the genus *Exiguobacterium*, the genus *Enterococcus*, the genus *Acinetobacter*, the genus *Turicibacter*, the genus *Klebsiella*, the genus *Lautropia*, the genus *Akkermansia*, the genus *Parabacteroides*, the genus *Rhizobium*, the genus *Actinomyces*, the genus *Veillonella*, the genus *Pseudomonas*, the genus *Rothia*, the genus *Dorea*, the genus *Streptococcus*, the genus *Haemophilus*, the genus *Enhydrobacter*, the genus *Rhodococcus*, the genus *Coprococcus*, the genus *Oscillospira*, the genus *Ruminococcus*, the genus *Bacteroides*, the genus *Corynebacterium*, the genus *Weissella*, the genus *Propionibacterium*, the genus *Lysinibacillus*, the genus *Stenotrophomonas*, the genus *Arthrobacter*, the genus *Comamonas*, the genus *Marinobacter*, the genus *Clostridium*, the genus *Planctomyces*, the genus *Luteolibacter*, the genus *Delftia*, the genus *Agrobacterium*, the genus *Rhodoplanes*, the genus *Gemmata*, the genus *Coprobacillus*, the genus *Arcobacter*, the genus *Helicobacter*, the genus *Candidatus Solibacter*, the genus *Methanosarcina*, the genus *Thermacetogenium*, the genus *Synechococcus*, the genus *Desulfomicrobium*, the genus *Chthoniobacter*, the genus *Aminobacterium*, the genus *Gallicola*, the genus *Anaeromyxobacter*, the genus *Muricauda*, and the genus *Candidatus Koribacter* was significantly different between Parkinson's disease patients and normal individuals (see Example 4).

From the above-described example results, it was confirmed that the identified distribution variables of the bacterial-derived extracellular vesicles were able to be effectively used to predict the occurrence of Parkinson's disease.

Hereinafter, the present invention will be described with reference to exemplary examples to aid in understanding of the present invention. However, these examples are provided only for illustrative purposes and are not intended to limit the scope of the present invention.

### [Examples]

### Example 1. Analysis of In Vivo Absorption, Distribution, and Excretion Patterns of Intestinal Bacteria and Bacteria-Derived Extracellular Vesicles

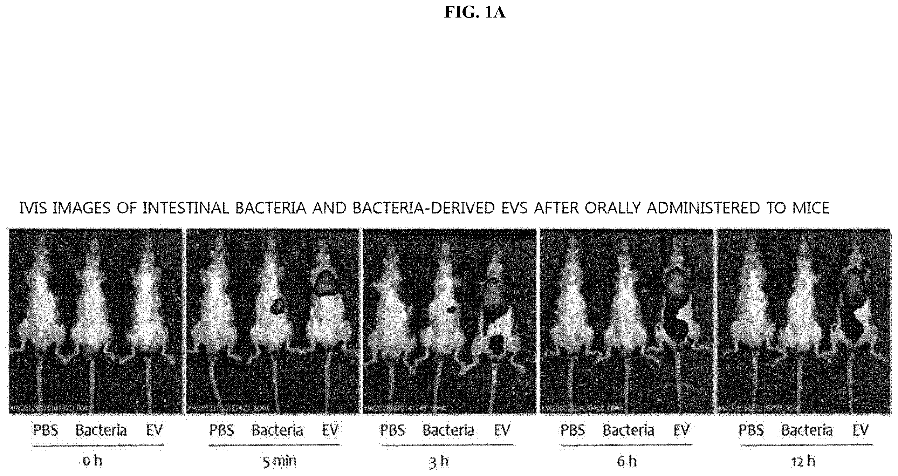

To evaluate whether intestinal bacteria and bacteria-derived extracellular vesicles are systematically absorbed through the gastrointestinal tract, an experiment was conducted using the following method. More particularly, 50 µg of each of intestinal bacteria and the bacteria-derived extracellular vesicles (EVs), labeled with fluorescence, were orally administered to the gastrointestinal tracts of mice, and fluorescence was measured at 0 h, and after 5 min, 3 h, 6 h, and 12 h. As a result of observing the entire images of mice, as illustrated in FIG. 1A, the bacteria were not systematically absorbed when administered, while the bacteria-derived EVs were systematically absorbed at 5 min after administration, and, at 3 h after administration, fluorescence was strongly observed in the bladder, from which it was confirmed that the EVs were excreted via the urinary system, and were present in the bodies up to 12 h after administration.

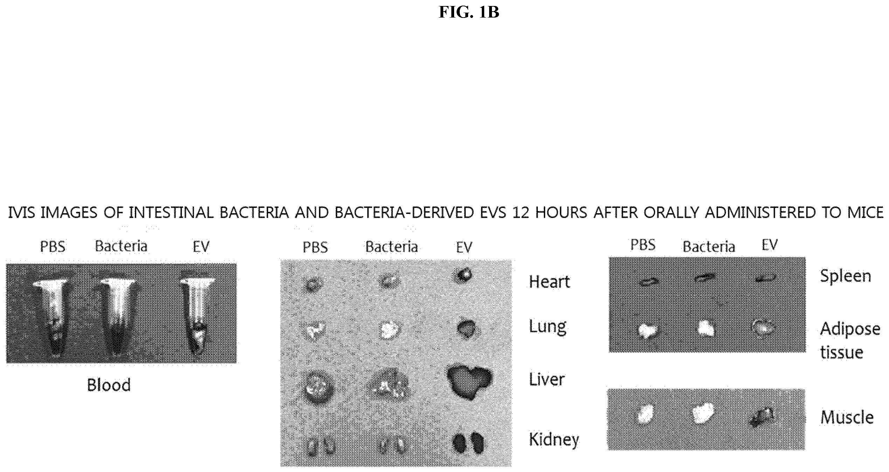

After intestinal bacteria and intestinal bacteria-derived extracellular vesicles were systematically absorbed, to evaluate a pattern of invasion of intestinal bacteria and the bacteria-derived EVs into various organs in the human body after being systematically absorbed, 50 µg of each of the bacteria and bacteria-derived EVs, labeled with fluorescence, were administered using the same method as that used above, and then, at 12 h after administration, blood, the heart, the lungs, the liver, the kidneys, the spleen, adipose tissue, and muscle were extracted from each mouse. As a result of observing fluorescence in the extracted tissues, as illustrated in FIG. 1B, it was confirmed that the intestinal bacteria were not absorbed into each organ, while the bacteria-derived EVs were distributed in the blood, heart, lungs, liver, kidneys, spleen, adipose tissue, and muscle.

### Example 2. Vesicle Isolation and DNA Extraction from Urine

To isolate extracellular vesicles and extract DNA, from urine, first, urine was added to a 10 ml tube and centrifuged at 3,500 x g and 4 °C for 10 min to precipitate a suspension, and only a supernatant was collected, which was then placed in a new 10 ml tube. The collected supernatant was filtered using a 0.22 µm filter to remove bacteria and impurities, and then placed in centripreigugal filters (50 kD) and centrifuged at 1500 x g and 4 °C for 15 min to discard materials with a smaller size than 50 kD, and then concentrated to 10 ml. Once again, bacteria and impurities were removed therefrom using a 0.22 µm filter, and then the resulting concentrate was subjected to ultra-high speed centrifugation at 150,000 x g and 4 °C for 3 hours by using a Type 90ti rotor to remove a supernatant, and the agglomerated pellet was dissolved with phosphate-buffered saline (PBS), thereby obtaining vesicles.

100 µl of the extracellular vesicles isolated from the urine according to the above-described method was boiled at 100 °C to allow the internal DNA to come out of the lipid and then cooled on ice. Next, the resulting vesicles were centrifuged at 10,000 x g and 4 °C for 30 minutes to remove the remaining suspension, only the supernatant was collected, and then the amount of DNA extracted was quantified using a NanoDrop sprectrophotometer. In addition, to verify whether bacteria-derived DNA was present in the extracted DNA, PCR was performed using 16s rDNA primers shown in Table 1 below.

**[Table 1]**

| Primer | | Sequence | SEQ ID NO. |
|---|---|---|---|
| 16S rDNA | 16S_V3_F | 5'-TCGTCGGCAGCGTC AGATGTGTATAAGAG ACAGCCTACGGGNGG CWGCAG-3' | 1 |
| | 16S_V4_R | 5'-GTCTCGTGGGCTCG GAGATGTGTATAAGA GACAGGACTACHVGG GTATCTAATCC-3' | 2 |

### Example 3. Metagenomic Analysis Using DNA Extracted from Urine

DNA was extracted using the same method as that used in Example 2, and then PCR was performed thereon using 16S rDNA primers shown in Table 1 to amplify DNA, followed by sequencing (Illumina MiSeq sequencer). The results were output as standard flowgram format (SFF) files, and the SFF files were converted into sequence files (.fasta) and nucleotide quality score files using GS FLX software (v2.9), and then credit rating for reads was identified, and portions with a window (20 bps) average base call accuracy of less than 99% (Phred score <20) were removed. After removing the low-quality portions, only reads having a length of 300 bps or more were used (Sickle version 1.33), and, for operational taxonomy unit (OTU) analysis, clustering was performed using UCLUST and USEARCH according to sequence similarity. In particular, clustering was performed based on sequence similarity values of 94% for genus, 90% for family, 85% for order, 80% for class, and 75% for phylum, and phylum, class, order, family, and genus levels of each OTU were classified, and bacteria with a sequence similarity of 97% or more were analyzed (QIIME) using 16S DNA sequence databases (108,453 sequences) of BLASTN and GreenGenes.

### Example 4. Parkinson's Disease Diagnostic Model Based on Metagenomic Analysis of Bacteria-Derived EVs Isolated from Urine

EVs were isolated from urine samples of 39 Parkinson's disease patients and 76 normal individuals, the two groups matched in age and gender, and then metagenomic sequencing was performed thereon using the method of Example 3. For the development of a diagnostic model, first, a strain exhibiting a p value of less than 0.05 between two groups in a t-test and a difference of two-fold or more between two groups was selected, and then an area under curve (AUC), sensitivity, and specificity, which are diagnostic performance indexes, were calculated by logistic regression analysis.

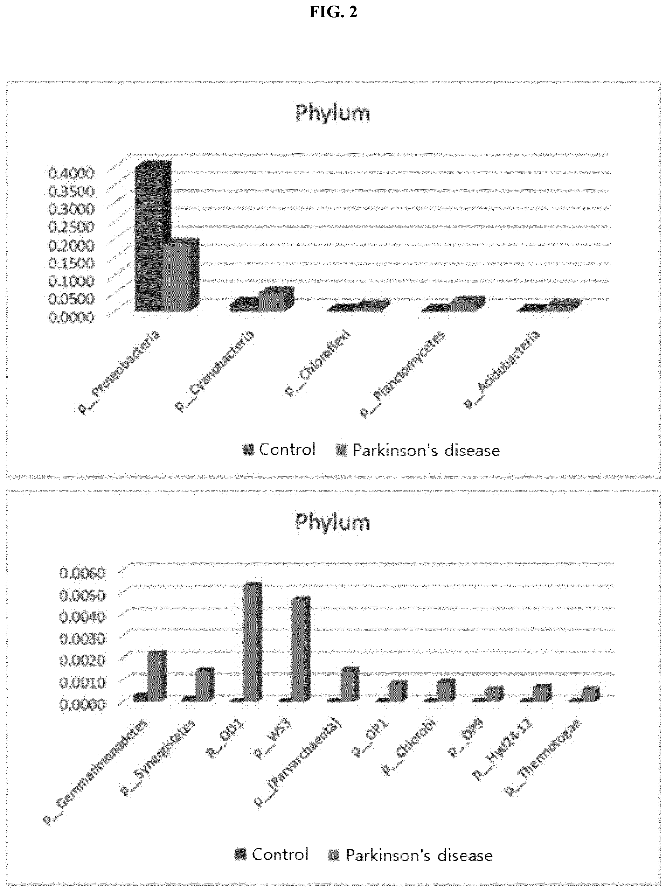

As a result of analyzing bacteria-derived EVs in urine at a phylum level, a diagnostic model developed using bacteria belonging to the phylum *Proteobacteria*, the phylum *Cyanobacteria*, the phylum *Gemmatimonadetes,* the phylum *Chloroflexi*, the phylum *Synergistetes*, the phylum *Acidobacteria*, the phylum *Planctomycetes*, the phylum OD1, the phylum WS3, the phylum *Parvarchaeota*, the phylum OP1, the phylum *Chlorobi*, the phylum OP9, the phylum Hyd24-12, and the phylum *Thermotogae* as a biomarker exhibited significant diagnostic performance for Parkinson's disease (see Table 2 and FIG. 2).

**[Table 2]**

| | Control | | Parkinson's disease | | t-test | | | | |
|---|---|---|---|---|---|---|---|---|---|
| Taxon | Mean | SD | Mean | SD | p-value | Ratio | AUC | Sensiti vity | Specifi city |
| p_Proteobacteria | 0.3993 | 0.1837 | 0.1836 | 0.0974 | 0.0000 | 0.46 | 0.95 | 0.86 | 0.92 |
| p_Cyanobacteria | 0.0191 | 0.0207 | 0.0486 | 0.0184 | 0.0000 | 2.55 | 0.87 | 0.86 | 0.54 |
| p_Gemmatimona detes | 0.0003 | 0.0010 | 0.0022 | 0.0021 | 0.0000 | 8.03 | 0.81 | 0.93 | 0.64 |
| p_Chloroflexi | 0.0010 | 0.0030 | 0.0116 | 0.0052 | 0.0000 | 11.79 | 0.95 | 0.95 | 0.87 |
| p_Synergistetes | 0.0001 | 0.0003 | 0.0014 | 0.0018 | 0.0001 | 15.89 | 0.81 | 0.96 | 0.49 |
| p_Acidobacteria | 0.0005 | 0.0012 | 0.0119 | 0.0049 | 0.0000 | 25.85 | 1.00 | 0.97 | 0.95 |
| p_Planctomycete s | 0.0006 | 0.0019 | 0.0222 | 0.0094 | 0.0000 | 37.99 | 1.00 | 0.97 | 0.92 |
| p_OD1 | 0.0000 | 0.0000 | 0.0053 | 0.0043 | 0.0000 | 1219.9 0 | 0.97 | 0.99 | 0.85 |
| p_WS3 | 0.0000 | 0.0000 | 0.0046 | 0.0027 | 0.0000 | | 1.00 | 1.00 | 1.00 |
| p_[Parvarchaeota ] | 0.0000 | 0.0000 | 0.0014 | 0.0025 | 0.0000 | | 0.95 | 1.00 | 0.85 |
| p_OP1 | 0.0000 | 0.0000 | 0.0008 | 0.0014 | 0.0000 | | 0.80 | 1.00 | 0.51 |
| p_Chlorobi | 0.0000 | 0.0000 | 0.0009 | 0.0012 | 0.0000 | | 0.79 | 1.00 | 0.54 |
| p_OP9 | 0.0000 | 0.0000 | 0.0005 | 0.0009 | 0.0000 | | 0.76 | 1.00 | 0.49 |
| p_Hyd24-12 | 0.0000 | 0.0000 | 0.0006 | 0.0012 | 0.0000 | | 0.75 | 1.00 | 0.44 |
| p_Thermotogae | 0.0000 | 0.0000 | 0.0005 | 0.0013 | 0.0005 | | 0.62 | 1.00 | 0.21 |

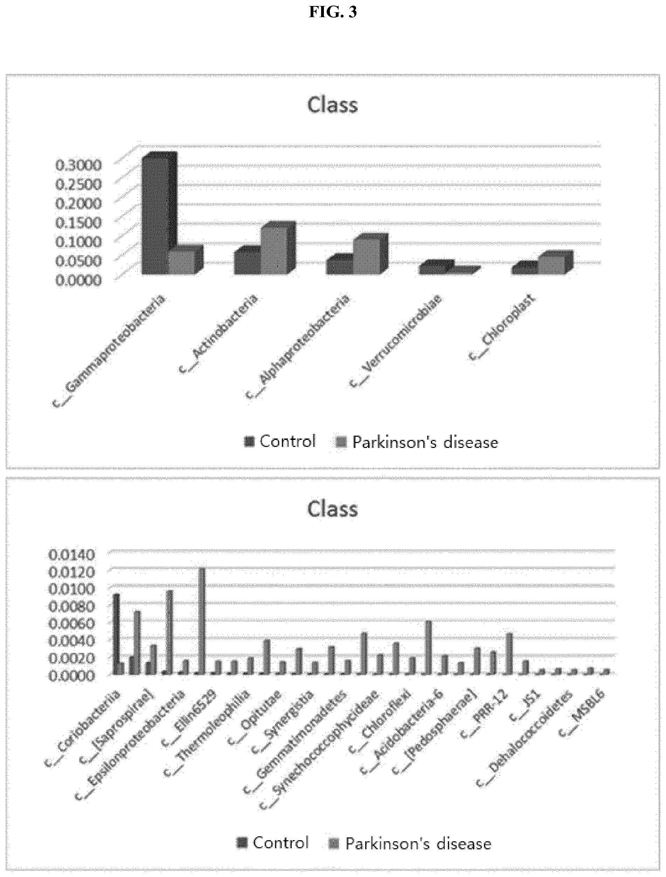

As a result of analyzing bacteria-derived EVs in urine at a class level, a diagnostic model developed using bacteria belonging to the class *Coriobacteriia*, the class *Gammaproteobacteria*, the class *Verrucomicrobiae*, the class *Actinobacteria*, the class *Alphaproteobacteria*, the class *Chloroplast*, the class *Saprospirae*, the class *Deltaproteobacteria*, the class *Epsilonproteobacteria*, the class Ellin6529, the class *Chloracidobacteria*, the class *Opitutae*, the class *Thermoleophilia*, the class *Synergistia*, the class *Gemmatimonadetes,* the class *Planctomycetia*, the class *Acidobacteriia*, the class *Solibacteres*, the class *Anaerolineae*, the class *Chloroflexi,* the class *Phycisphaerae*, the class *Synechococcophycideae*, the class TM7-1, the class *Acidimicrobiia*, the class *Acidobacteria*-6, the class *Spartobacteria*, the class ABY1, the class *Pedosphaerae*, the class ZB2, the class PRR-12, the class *Ktedonobacteria*, the class JS1, the class WM88, the class *Dehalococcoidetes*, the class SAR202, and the class MSBL6 as a biomarker exhibited significant diagnostic performance for Parkinson's disease (see Table 3 and FIG. 3).

**[Table 3]**

| | Control | | Parkinson's disease | | t-test | | | | |
|---|---|---|---|---|---|---|---|---|---|
| Taxon | Mean | SD | Mean | SD | p-value | Ratio | AUC | Sensiti vity | Specificity |
| c_Coriobacteriia | 0.009 2 | 0.008 0 | 0.001 3 | 0.001 5 | 0.000 0 | 0.14 | 0.85 | 0.78 | 0.72 |
| c_Gammaproteobact eria | 0.299 9 | 0.171 6 | 0.059 3 | 0.110 3 | 0.000 0 | 0.20 | 0.96 | 0.96 | 0.97 |
| c_Verrucomicrobiae | 0.021 8 | 0.027 2 | 0.005 2 | 0.004 4 | 0.000 0 | 0.24 | 0.73 | 0.79 | 0.36 |
| c_Actinobacteria | 0.057 9 | 0.032 0 | 0.120 2 | 0.055 3 | 0.000 0 | 2.08 | 0.91 | 0.88 | 0.74 |
| c_Alphaproteobacter ia | 0.037 2 | 0.043 4 | 0.089 7 | 0.024 4 | 0.000 0 | 2.41 | 0.94 | 0.96 | 0.79 |
| c_Chloroplast | 0.018 5 | 0.020 2 | 0.045 4 | 0.018 1 | 0.000 0 | 2.46 | 0.86 | 0.86 | 0.51 |
| c_[Saprospirae] | 0.001 3 | 0.003 0 | 0.003 3 | 0.003 0 | 0.001 5 | 2.46 | 0.75 | 0.92 | 0.23 |
| c_Deltaproteobacteri a | 0.002 0 | 0.003 6 | 0.007 2 | 0.004 0 | 0.000 0 | 3.61 | 0.85 | 0.89 | 0.51 |
| c_Epsilonproteobact eria | 0.000 3 | 0.001 0 | 0.001 6 | 0.002 1 | 0.000 7 | 5.52 | 0.74 | 0.95 | 0.44 |
| c_Ellin6529 | 0.000 2 | 0.001 6 | 0.001 5 | 0.001 6 | 0.000 1 | 7.88 | 0.89 | 0.99 | 0.49 |
| c_[Chloracidobacteri a] | 0.000 1 | 0.000 8 | 0.001 5 | 0.001 5 | 0.000 0 | 10.81 | 0.87 | 0.97 | 0.54 |
| c_Opitutae | 0.000 1 | 0.000 6 | 0.001 4 | 0.002 0 | 0.000 2 | 12.65 | 0.75 | 0.95 | 0.44 |
| c_Thermoleophilia | 0.000 1 | 0.000 6 | 0.001 9 | 0.001 8 | 0.000 0 | 13.97 | 0.86 | 0.96 | 0.64 |
| c_Synergistia | 0.000 1 | 0.000 3 | 0.001 4 | 0.001 8 | 0.000 1 | 15.89 | 0.81 | 0.96 | 0.49 |
| c_Gemmatimonadet es | 0.000 1 | 0.000 4 | 0.001 6 | 0.001 5 | 0.000 0 | 21.15 | 0.82 | 0.96 | 0.64 |
| c_Planctomycetia | 0.000 4 | 0.001 5 | 0.009 6 | 0.004 9 | 0.000 0 | 25.27 | 0.98 | 0.97 | 0.92 |
| c_Acidobacteriia | 0.000 1 | 0.000 5 | 0.002 9 | 0.002 4 | 0.000 0 | 30.81 | 0.92 | 0.96 | 0.77 |
| c_Solibacteres | 0.000 1 | 0.000 6 | 0.003 9 | 0.003 6 | 0.000 0 | 33.37 | 0.92 | 0.96 | 0.74 |
| c_Anaerolineae | 0.000 1 | 0.000 4 | 0.003 2 | 0.002 9 | 0.000 0 | 40.61 | 0.89 | 0.97 | 0.69 |
| c_Chloroflexi | 0.000 0 | 0.000 2 | 0.001 9 | 0.001 8 | 0.000 0 | 55.61 | 0.84 | 0.97 | 0.59 |
| c_Phycisphaerae | 0.000 2 | 0.001 3 | 0.012 1 | 0.007 7 | 0.000 0 | 59.10 | 0.96 | 0.99 | 0.85 |
| c_Synechococcophy cideae | 0.000 0 | 0.000 3 | 0.002 2 | 0.003 0 | 0.000 0 | 61.69 | 0.86 | 0.99 | 0.49 |
| c_TM7-1 | 0.000 1 | 0.000 3 | 0.004 7 | 0.004 0 | 0.000 0 | 89.44 | 0.94 | 0.99 | 0.77 |
| c_Acidimicrobiia | 0.000 0 | 0.000 3 | 0.003 6 | 0.002 5 | 0.000 0 | 100.35 | 0.97 | 0.99 | 0.85 |
| c_Acidobacteria-6 | 0.000 0 | 0.000 1 | 0.002 2 | 0.002 1 | 0.000 0 | 140.15 | 0.88 | 0.99 | 0.69 |
| c_[Spartobacteria] | 0.000 0 | 0.000 2 | 0.006 1 | 0.003 8 | 0.000 0 | 201.78 | 0.99 | 0.99 | 0.92 |
| c_ABY1 | 0.000 0 | 0.000 0 | 0.001 3 | 0.002 2 | 0.000 8 | 324.38 | 0.78 | 0.99 | 0.44 |
| c_[Pedosphaerae] | 0.000 0 | 0.000 0 | 0.003 0 | 0.001 9 | 0.000 0 | 2033.1 9 | 1.00 | 1.00 | 1.00 |
| c_ZB2 | 0.000 0 | 0.000 0 | 0.002 6 | 0.003 4 | 0.000 0 | 10532. 52 | 0.90 | 0.99 | 0.72 |
| c_PRR-12 | 0.000 0 | 0.000 0 | 0.004 6 | 0.002 7 | 0.000 0 | | 1.00 | 1.00 | 1.00 |
| c_Ktedonobacteria | 0.000 0 | 0.000 0 | 0.001 5 | 0.001 9 | 0.000 0 | | 0.88 | 1.00 | 0.74 |
| c_JS1 | 0.000 0 | 0.000 0 | 0.000 5 | 0.000 9 | 0.000 0 | | 0.76 | 1.00 | 0.49 |
| c_WM88 | 0.000 0 | 0.000 0 | 0.000 6 | 0.001 2 | 0.000 0 | | 0.75 | 1.00 | 0.44 |
| c_Dehalococcoidetes | 0.000 0 | 0.000 0 | 0.000 5 | 0.001 2 | 0.000 1 | | 0.73 | 1.00 | 0.41 |
| c_SAR202 | 0.000 0 | 0.000 0 | 0.000 7 | 0.001 5 | 0.000 1 | | 0.72 | 1.00 | 0.36 |
| c_MSBL6 | 0.000 0 | 0.000 0 | 0.000 5 | 0.001 1 | 0.000 0 | | 0.70 | 1.00 | 0.38 |

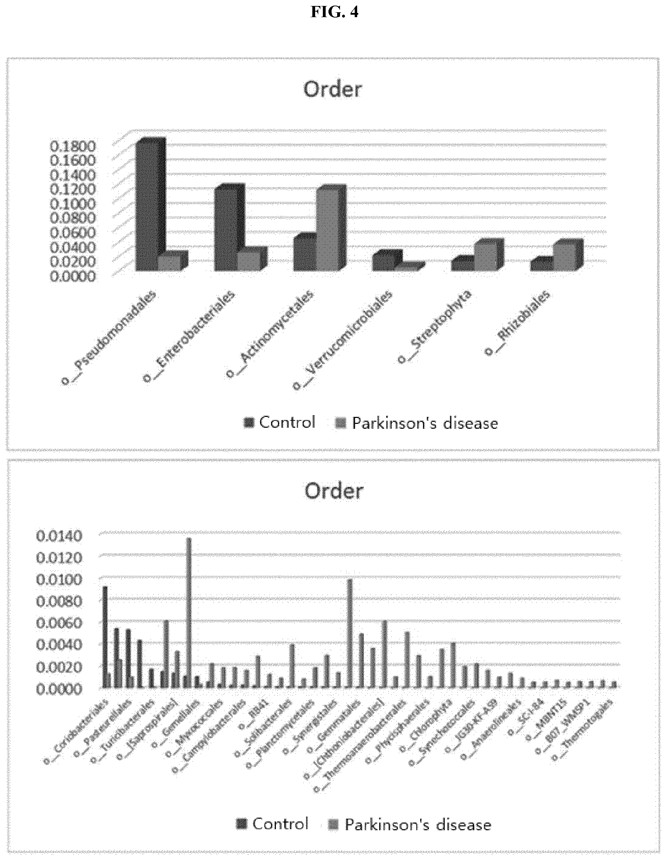

As a result of analyzing bacteria-derived EVs in urine at an order level, a diagnostic model developed using bacteria belonging to the order RF39, the order *Turicibacterales*, the order *Pseudomonadales*, the order *Coriobacteriales*, the order *Pasteurellales*, the order *Enterobacteriales*, the order *Verrucomicrobiales*, the order *Gemellales*, the order *Neisseriales*, the order *Saprospirales*, the order *Actinomycetales*, the order *Streptophyta*, the order *Rhizobiales*, the order *Rhodospirillales*, the order *Xanthomonadales*, the order *Myxococcales*, the order *Campylobacterales*, the order *Desulfovibrionales*, the order *Solirubrobacterales*, the order *Opitutales*, the order RB41, the order *Rickettsiales*, the order *Pirellulales*, the order *Synergistales*, the order *Planctomycetales*, the order *Acidobacteriales*, the order *Solibacterales*, the order *Gaiellales*, the order *Gemmatales*, the order *Acidimicrobiales*, the order WD2101, the order *Chthoniobacterales*, the order *Thermoanaerobacterales*, the order *Pedosphaerales*, the order *Phycisphaerales*, the order Sediment-1, the order *Chlorophyta*, the order iii1-15, the order *Synechococcales*, the order *Roseiflexales*, the order JG30-KF-AS9, the order Ellin329, the order *Anaerolineales*, the order Ellin5290, the order SC-I-84, the order *Cryptophyta*, the order MBNT15, the order envOPS12, the order B07_WMSP1, the order UA01, and the order *Thermotogales* as a biomarker exhibited significant diagnostic performance for Parkinson's disease (see Table 4 and FIG. 4).

**[Table 4]**

| | Control | | Parkinson's disease | | t-test | | | | |
|---|---|---|---|---|---|---|---|---|---|
| Taxon | Mean | SD | Mean | SD | p-value | Ratio | AUC | Sensiti vity | Specificity |
| o_RF39 | 0.0043 | 0.0078 | 0.0000 | 0.0000 | 0.0000 | 0.00 | 0.93 | 0.86 | 0.97 |
| o_Turicibacterale s | 0.0017 | 0.0025 | 0.0001 | 0.0004 | 0.0000 | 0.04 | 0.90 | 0.74 | 0.97 |
| o_Pseudomonadal es | 0.1765 | 0.1640 | 0.0206 | 0.0091 | 0.0000 | 0.12 | 1.00 | 0.97 | 1.00 |
| o_Coriobacteriale s | 0.0092 | 0.0080 | 0.0013 | 0.0015 | 0.0000 | 0.14 | 0.93 | 0.87 | 0.92 |
| o_Pasteurellales | 0.0053 | 0.0061 | 0.0010 | 0.0013 | 0.0000 | 0.19 | 0.91 | 0.82 | 0.79 |
| o_Enterobacterial es | 0.1129 | 0.0780 | 0.0260 | 0.1144 | 0.0001 | 0.23 | 0.98 | 0.93 | 0.97 |
| o_Verrucomicrob iales | 0.0218 | 0.0272 | 0.0052 | 0.0044 | 0.0000 | 0.24 | 0.90 | 0.82 | 0.90 |
| o_Gemellales | 0.0010 | 0.0017 | 0.0003 | 0.0008 | 0.0036 | 0.31 | 0.83 | 0.74 | 0.74 |
| o_Neisseriales | 0.0054 | 0.0077 | 0.0026 | 0.0026 | 0.0050 | 0.48 | 0.82 | 0.75 | 0.62 |
| o_[Saprospirales] | 0.0013 | 0.0030 | 0.0033 | 0.0030 | 0.0015 | 2.46 | 0.88 | 0.89 | 0.62 |
| o_Actinomycetale s | 0.0452 | 0.0290 | 0.1116 | 0.0549 | 0.0000 | 2.47 | 0.95 | 0.89 | 0.90 |
| o_Streptophyta | 0.0142 | 0.0184 | 0.0375 | 0.0152 | 0.0000 | 2.63 | 0.93 | 0.91 | 0.82 |
| o_Rhizobiales | 0.0128 | 0.0111 | 0.0368 | 0.0148 | 0.0000 | 2.88 | 0.95 | 0.96 | 0.79 |
| o_Rhodospirillale s | 0.0005 | 0.0017 | 0.0022 | 0.0022 | 0.0001 | 4.04 | 0.90 | 0.93 | 0.59 |
| o_Xanthomonada les | 0.0015 | 0.0022 | 0.0061 | 0.0036 | 0.0000 | 4.12 | 0.94 | 0.89 | 0.69 |
| o_Myxococcales | 0.0003 | 0.0013 | 0.0018 | 0.0022 | 0.0004 | 5.24 | 0.89 | 0.87 | 0.64 |
| o_Campylobacter ales | 0.0003 | 0.0010 | 0.0016 | 0.0021 | 0.0007 | 5.52 | 0.88 | 0.91 | 0.56 |
| o_Desulfovibrion ales | 0.0003 | 0.0007 | 0.0018 | 0.0022 | 0.0001 | 6.33 | 0.89 | 0.84 | 0.67 |
| o_Solirubrobacter ales | 0.0001 | 0.0006 | 0.0009 | 0.0014 | 0.0027 | 7.42 | 0.85 | 0.87 | 0.62 |
| ο_Opitutales | 0.0001 | 0.0005 | 0.0008 | 0.0015 | 0.0070 | 7.54 | 0.83 | 0.91 | 0.38 |
| o_RB41 | 0.0001 | 0.0008 | 0.0012 | 0.0014 | 0.0000 | 9.27 | 0.98 | 0.99 | 0.79 |
| o_Rickettsiales | 0.0011 | 0.0019 | 0.0136 | 0.0086 | 0.0000 | 12.59 | 0.98 | 0.97 | 0.90 |
| o_Pirellulales | 0.0002 | 0.0014 | 0.0029 | 0.0023 | 0.0000 | 13.12 | 0.93 | 0.99 | 0.72 |
| o_Synergistales | 0.0001 | 0.0003 | 0.0014 | 0.0018 | 0.0001 | 15.89 | 0.91 | 0.97 | 0.59 |
| ο_Planctomycetal es | 0.0001 | 0.0006 | 0.0018 | 0.0021 | 0.0000 | 18.78 | 0.91 | 0.95 | 0.59 |
| o_Acidobacteriale s | 0.0001 | 0.0005 | 0.0029 | 0.0024 | 0.0000 | 30.81 | 0.96 | 0.97 | 0.82 |
| o_Solibacterales | 0.0001 | 0.0006 | 0.0039 | 0.0036 | 0.0000 | 33.37 | 0.96 | 0.99 | 0.74 |
| o_Gaiellales | 0.0000 | 0.0001 | 0.0010 | 0.0013 | 0.0000 | 61.75 | 0.91 | 0.99 | 0.54 |
| o_Gemmatales | 0.0001 | 0.0003 | 0.0049 | 0.0036 | 0.0000 | 76.01 | 0.97 | 0.99 | 0.79 |
| o_Acidimicrobial es | 0.0000 | 0.0003 | 0.0036 | 0.0025 | 0.0000 | 100.35 | 1.00 | 0.99 | 0.90 |
| o_WD2101 | 0.0001 | 0.0004 | 0.0098 | 0.0067 | 0.0000 | 153.20 | 0.99 | 0.99 | 0.92 |
| o_[Chthoniobacte rales] | 0.0000 | 0.0002 | 0.0061 | 0.0038 | 0.0000 | 201.78 | 1.00 | 1.00 | 1.00 |
| o_Thermoanaerob | 0.0000 | 0.0001 | 0.0050 | 0.0028 | 0.0000 | 337.41 | 0.99 | 0.99 | 0.92 |
| acterales | | | | | | | | | |
| o_[Pedosphaerale s] | 0.0000 | 0.0000 | 0.0029 | 0.0019 | 0.0000 | 1980.86 | 1.00 | 1.00 | 1.00 |
| o_Phycisphaerale s | 0.0000 | 0.0000 | 0.0010 | 0.0022 | 0.0060 | 3096.39 | 0.89 | 0.99 | 0.49 |
| o_Sediment-1 | 0.0000 | 0.0000 | 0.0035 | 0.0023 | 0.0000 | | 1.00 | 1.00 | 0.97 |
| o_Chlorophyta | 0.0000 | 0.0000 | 0.0041 | 0.0047 | 0.0000 | | 0.98 | 1.00 | 0.92 |
| o_iii1-15 | 0.0000 | 0.0000 | 0.0019 | 0.0021 | 0.0000 | | 0.97 | 1.00 | 0.82 |
| o_Synechococcal es | 0.0000 | 0.0000 | 0.0022 | 0.0029 | 0.0000 | | 0.95 | 1.00 | 0.79 |
| o_[Roseiflexales] | 0.0000 | 0.0000 | 0.0016 | 0.0016 | 0.0000 | | 0.95 | 1.00 | 0.69 |
| o_JG30-KF-AS9 | 0.0000 | 0.0000 | 0.0010 | 0.0013 | 0.0000 | | 0.93 | 1.00 | 0.67 |
| o_Ellin329 | 0.0000 | 0.0000 | 0.0013 | 0.0026 | 0.0000 | | 0.93 | 1.00 | 0.59 |
| o_Anaerolineales | 0.0000 | 0.0000 | 0.0009 | 0.0017 | 0.0000 | | 0.89 | 0.97 | 0.49 |
| o_Ellin5290 | 0.0000 | 0.0000 | 0.0005 | 0.0011 | 0.0000 | | 0.89 | 0.92 | 0.56 |
| o_SC-I-84 | 0.0000 | 0.0000 | 0.0005 | 0.0010 | 0.0000 | | 0.89 | 1.00 | 0.46 |
| ο_Cryptophyta | 0.0000 | 0.0000 | 0.0007 | 0.0013 | 0.0000 | | 0.88 | 0.97 | 0.46 |
| o_MBNT15 | 0.0000 | 0.0000 | 0.0005 | 0.0011 | 0.0001 | | 0.88 | 0.92 | 0.59 |
| o_envOPS12 | 0.0000 | 0.0000 | 0.0006 | 0.0011 | 0.0000 | | 0.87 | 1.00 | 0.41 |
| o_B07_WMSP1 | 0.0000 | 0.0000 | 0.0006 | 0.0013 | 0.0003 | | 0.87 | 0.92 | 0.46 |
| o_UA01 | 0.0000 | 0.0000 | 0.0007 | 0.0020 | 0.0036 | | 0.86 | 0.97 | 0.38 |
| o_Thermotogales | 0.0000 | 0.0000 | 0.0005 | 0.0013 | 0.0005 | | 0.83 | 0.89 | 0.44 |

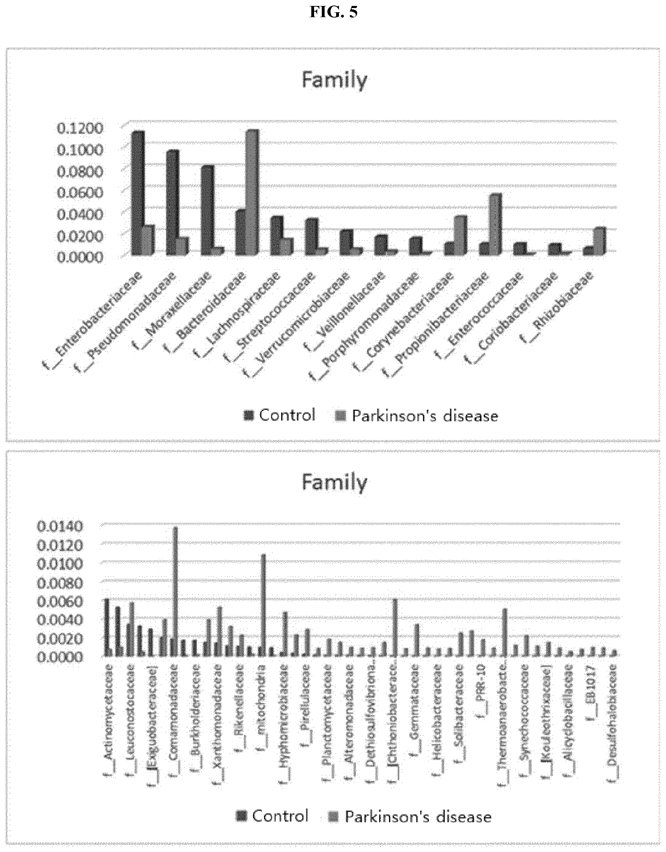

As a result of analyzing bacteria-derived EVs in urine at a family level, a diagnostic model developed using bacteria belonging to the family *Exiguobacteraceae*, the family *Enterococcaceae*, the family *Turicibacteraceae*, the family *Mogibacteriaceae*, the family *Moraxellaceae*, the family *Porphyromonadaceae*, the family *Burkholderiaceae*, the family *Actinomycetaceae*, the family *Coriobacteriaceae*, the family *Methylobacteriaceae*, the family *Streptococcaceae*, the family *Pseudomonadaceae*, the family *Pasteurellaceae*, the family *Veillonellaceae*, the family *Peptostreptococcaceae*, the family *Enterobacteriaceae*, the family *Verrucomicrobiaceae*, the family *Lachnospiraceae*, the family *Leuconostocaceae*, the family *Bradyrhizobiaceae*, the family *Rikenellaceae,* the family *Tissierellaceae,* the family *Bacteroidaceae,* the family *Chitinophagaceae,* the family *Corynebacteriaceae,* the family *Xanthomonadaceae,* the family *Rhizobiaceae,* the family *Propionibacteriaceae,* the family *Desulfobacteraceae,* the family *Barnesiellaceae,* the family *Comamonadaceae,* the family *mitochondria,* the family *Hyphomicrobiaceae,* the family *Alteromonadaceae,* the family *Sinobacteraceae,* the family *Pirellulaceae,* the family *Dethiosulfovibrionaceae,* the family *Acidobacteriaceae,* the family *Planctomycetaceae,* the family *Isosphaeraceae,* the family *Gaiellaceae,* the family *Koribacteraceae,* the family *Helicobacteraceae,* the family *Chthoniobacteraceae,* the family *Gemmataceae,* the family C111, the family *Solibacteraceae,* the family *Pelagibacteraceae,* the family PRR-10, the family Ellin515, the family *Thermoanaerobacteraceae,* the family *Methanoregulaceae,* the family *Synechococcaceae,* the family *Desulfomicrobiaceae,* the family *Kouleothrixaceae,* the family OCS155, the family *Alicyclobacillaceae,* the family *Myxococcaceae,* the family EB1017, the family *Anaerolinaceae,* and the family *Desulfohalobiaceae* as a biomarker exhibited significant diagnostic performance for Parkinson's disease (see Table 5 and FIG. 5).

**[Table 5]**

| | Control | | Parkinson's disease | | t-test | | | | |
|---|---|---|---|---|---|---|---|---|---|
| Taxon | Mean | SD | Mean | SD | p-value | Ratio | AUC | Sensiti vity | Specif icity |
| f_[Exiguobacterac eae] | 0.0029 | 0.0064 | 0.0000 | 0.0000 | 0.0002 | 0.00 | 0.73 | 0.87 | 0.21 |
| f_Enterococcaceae | 0.0100 | 0.0106 | 0.0003 | 0.0005 | 0.0000 | 0.03 | 0.92 | 0.87 | 0.90 |
| f_Turicibacteracea e | 0.0017 | 0.0025 | 0.0001 | 0.0004 | 0.0000 | 0.04 | 0.75 | 0.99 | 0.08 |
| f_[Mogibacteriace ae] | 0.0009 | 0.0014 | 0.0000 | 0.0003 | 0.0000 | 0.05 | 0.75 | 0.84 | 0.26 |
| f_Moraxellaceae | 0.0810 | 0.1062 | 0.0058 | 0.0041 | 0.0000 | 0.07 | 0.97 | 0.93 | 0.92 |
| f_Porphyromonada ceae | 0.0151 | 0.0169 | 0.0012 | 0.0017 | 0.0000 | 0.08 | 0.85 | 0.82 | 0.74 |
| f_Burkholderiacea e | 0.0017 | 0.0028 | 0.0001 | 0.0004 | 0.0000 | 0.09 | 0.79 | 0.91 | 0.44 |
| f_Actinomycetac e | 0.0061 | 0.0095 | 0.0007 | 0.0010 | 0.0000 | 0.12 | 0.80 | 0.78 | 0.67 |
| f_Coriobacteriacea e | 0.0092 | 0.0080 | 0.0013 | 0.0015 | 0.0000 | 0.14 | 0.85 | 0.78 | 0.72 |
| f_Methylobacteria ceae | 0.0032 | 0.0056 | 0.0005 | 0.0009 | 0.0001 | 0.15 | 0.71 | 0.95 | 0.05 |
| f_Streptococcaceae | 0.0322 | 0.0227 | 0.0050 | 0.0035 | 0.0000 | 0.15 | 0.90 | 0.82 | 0.87 |
| f_Pseudomonadace ae | 0.0954 | 0.0850 | 0.0148 | 0.0074 | 0.0000 | 0.15 | 0.98 | 0.95 | 0.90 |
| f_Pasteurellaceae | 0.0053 | 0.0061 | 0.0010 | 0.0013 | 0.0000 | 0.19 | 0.75 | 0.72 | 0.59 |
| f_Veillonellaceae | 0.0169 | 0.0227 | 0.0032 | 0.0026 | 0.0000 | 0.19 | 0.79 | 0.78 | 0.69 |
| f_Peptostreptococc aceae | 0.0010 | 0.0015 | 0.0002 | 0.0006 | 0.0001 | 0.20 | 0.73 | 0.97 | 0.05 |
| f_Enterobacteriace ae | 0.1129 | 0.0780 | 0.0260 | 0.1144 | 0.0001 | 0.23 | 0.94 | 0.95 | 0.95 |
| f_Verrucomicrobia ceae | 0.0218 | 0.0272 | 0.0052 | 0.0044 | 0.0000 | 0.24 | 0.73 | 0.79 | 0.36 |
| f_Lachnospiraceae | 0.0343 | 0.0180 | 0.0140 | 0.0071 | 0.0000 | 0.41 | 0.89 | 0.88 | 0.74 |
| f_Leuconostocacea e | 0.0034 | 0.0039 | 0.0057 | 0.0035 | 0.0030 | 1.67 | 0.72 | 0.93 | 0.13 |
| f_Bradyrhizobiace ae | 0.0020 | 0.0050 | 0.0039 | 0.0034 | 0.0170 | 1.97 | 0.74 | 0.96 | 0.10 |
| f_Rikenellaceae | 0.0011 | 0.0021 | 0.0022 | 0.0023 | 0.0069 | 2.11 | 0.74 | 0.93 | 0.13 |
| f_[Tissierellaceae] | 0.0015 | 0.0025 | 0.0039 | 0.0028 | 0.0000 | 2.63 | 0.78 | 0.89 | 0.38 |
| f_Bacteroidaceae | 0.0405 | 0.0357 | 0.1142 | 0.0288 | 0.0000 | 2.82 | 0.94 | 0.95 | 0.87 |
| f_Chitinophagacea e | 0.0011 | 0.0021 | 0.0032 | 0.0031 | 0.0004 | 2.89 | 0.76 | 0.93 | 0.31 |
| f_Corynebacteriac eae | 0.0103 | 0.0104 | 0.0348 | 0.0154 | 0.0000 | 3.37 | 0.92 | 0.89 | 0.72 |
| f_Xanthomonadac eae | 0.0014 | 0.0022 | 0.0052 | 0.0034 | 0.0000 | 3.70 | 0.85 | 0.86 | 0.54 |
| f_Rhizobiaceae | 0.0063 | 0.0064 | 0.0240 | 0.0107 | 0.0000 | 3.80 | 0.93 | 0.95 | 0.72 |
| f_Propionibacteria ceae | 0.0101 | 0.0087 | 0.0550 | 0.0434 | 0.0000 | 5.45 | 0.98 | 0.96 | 0.92 |
| f_Desulfobacterace ae | 0.0001 | 0.0007 | 0.0008 | 0.0013 | 0.0025 | 6.30 | 0.71 | 0.96 | 0.26 |
| f_[Bamesiellaceae] | 0.0003 | 0.0009 | 0.0023 | 0.0023 | 0.0000 | 6.56 | 0.81 | 0.93 | 0.51 |
| f_Comamonadacea e | 0.0019 | 0.0026 | 0.0137 | 0.0076 | 0.0000 | 7.40 | 0.98 | 0.96 | 0.79 |
| f _mitochondria | 0.0010 | 0.0018 | 0.0108 | 0.0073 | 0.0000 | 11.19 | 0.95 | 0.97 | 0.79 |
| f_Hyphomicrobiac eae | 0.0004 | 0.0016 | 0.0047 | 0.0046 | 0.0000 | 11.33 | 0.86 | 0.96 | 0.51 |
| f_Alteromonadace ae | 0.0001 | 0.0005 | 0.0010 | 0.0014 | 0.0008 | 12.22 | 0.77 | 0.97 | 0.36 |
| f_Sinobacteraceae | 0.0001 | 0.0003 | 0.0009 | 0.0014 | 0.0008 | 12.62 | 0.73 | 0.96 | 0.38 |
| f_Pirellulaceae | 0.0002 | 0.0014 | 0.0029 | 0.0023 | 0.0000 | 13.12 | 0.90 | 0.97 | 0.69 |
| f_Dethiosulfovibri onaceae | 0.0001 | 0.0003 | 0.0009 | 0.0014 | 0.0004 | 15.62 | 0.76 | 0.96 | 0.36 |
| f_Acidobactenacea e | 0.0001 | 0.0005 | 0.0015 | 0.0020 | 0.0001 | 17.35 | 0.79 | 0.97 | 0.46 |
| f_Planctomycetace ae | 0.0001 | 0.0006 | 0.0018 | 0.0021 | 0.0000 | 18.78 | 0.82 | 0.97 | 0.49 |
| f_Isosphaeraceae | 0.0001 | 0.0003 | 0.0015 | 0.0019 | 0.0000 | 28.59 | 0.82 | 0.97 | 0.44 |
| f_Gaiellaceae | 0.0000 | 0.0001 | 0.0008 | 0.0010 | 0.0000 | 48.28 | 0.76 | 0.97 | 0.46 |
| f_Koribacteraceae | 0.0000 | 0.0001 | 0.0009 | 0.0015 | 0.0006 | 91.19 | 0.71 | 0.99 | 0.33 |
| f_Helicobacteracea e | 0.0000 | 0.0001 | 0.0008 | 0.0013 | 0.0015 | 114.37 | 0.84 | 0.99 | 0.41 |
| f_[Chthoni obactera ceae] | 0.0000 | 0.0002 | 0.0061 | 0.0038 | 0.0000 | 201.78 | 0.99 | 0.99 | 0.92 |
| f_Gemmataceae | 0.0000 | 0.0001 | 0.0034 | 0.0034 | 0.0000 | 273.92 | 0.91 | 0.99 | 0.69 |
| f_C111 | 0.0000 | 0.0000 | 0.0008 | 0.0013 | 0.0005 | 964.09 | 0.73 | 0.99 | 0.33 |
| f_Solibacteraceae | 0.0000 | 0.0000 | 0.0025 | 0.0024 | 0.0000 | 13883. 24 | 0.95 | 0.99 | 0.90 |
| f_Pelagibacteracea e | 0.0000 | 0.0000 | 0.0027 | 0.0033 | 0.0000 | | 0.97 | 1.00 | 0.92 |
| f_PRR-10 | 0.0000 | 0.0000 | 0.0018 | 0.0017 | 0.0000 | | 0.96 | 1.00 | 0.90 |
| f_Ellin515 | 0.0000 | 0.0000 | 0.0009 | 0.0008 | 0.0000 | | 0.95 | 1.00 | 0.85 |
| f_Thermoanaeroba cteraceae | 0.0000 | 0.0000 | 0.0050 | 0.0028 | 0.0000 | | 0.93 | 0.91 | 0.95 |
| f_Methanoregulace ae | 0. 0000 | 0.0000 | 0. 0012 | 0.0019 | 0. 0000 | | 0.92 | 1.00 | 0.82 |
| f_Synechococcace ae | 0.0000 | 0.0000 | 0.0022 | 0.0029 | 0.0000 | | 0.91 | 1.00 | 0.79 |
| f_Desulfomicrobia ceae | 0.0000 | 0.0000 | 0.0011 | 0.0014 | 0.0000 | | 0.88 | 1.00 | 0.69 |
| f_[Kouleothrixacea e] | 0.0000 | 0.0000 | 0.0015 | 0.0015 | 0.0000 | | 0.86 | 1.00 | 0.67 |
| f_OCS155 | 0.0000 | 0.0000 | 0.0009 | 0.0015 | 0.0000 | | 0.84 | 1.00 | 0.62 |
| f_Alicyclobacillac | 0.0000 | 0.0000 | 0.0005 | 0.0009 | 0.0000 | | 0.79 | 1.00 | 0.49 |
| eae | | | | | | | | | |
| f_Myxococcaceae | 0.0000 | 0.0000 | 0.0007 | 0.0012 | 0.0000 | | 0.76 | 1.00 | 0.46 |
| f_EB1017 | 0.0000 | 0.0000 | 0.0009 | 0.0015 | 0.0000 | | 0.76 | 1.00 | 0.51 |
| f_Anaerolinaceae | 0.0000 | 0.0000 | 0.0009 | 0.0017 | 0.0000 | | 0.74 | 1.00 | 0.44 |
| f_Desulfohalobiac eae | 0.0000 | 0.0000 | 0.0006 | 0.0014 | 0.0002 | | 0.73 | 1.00 | 0.41 |

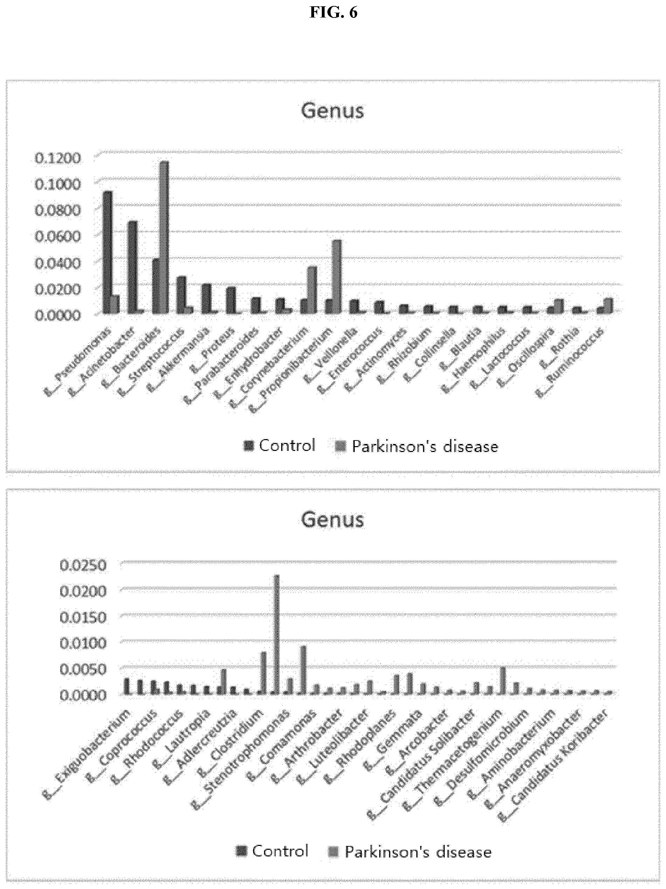

As a result of analyzing bacteria-derived EVs in urine at a genus level, a diagnostic model developed using bacteria belonging to the genus *Collinsella,* the genus *Adlercreutzia,* the genus SMB53, the genus *Proteus,* the genus *Exiguobacterium,* the genus *Enterococcus,* the genus *Acinetobacter,* the genus *Turicibacter,* the genus *Klebsiella,* the genus *Lautropia,* the genus *Akkermansia,* the genus *Parabacteroides,* the genus *Rhizobium,* the genus *Actinomyces,* the genus *Lactococcus,* the genus *Blautia,* the genus *Veillonella,* the genus *Pseudomonas,* the genus *Rothia,* the genus *Dorea,* the genus *Streptococcus,* the genus *Haemophilus,* the genus *Enhydrobacter,* the genus *Rhodococcus,* the genus *Coprococcus,* the genus *Oscillospira,* the genus *Ruminococcus,* the genus *Bacteroides,* the genus *Corynebacterium,* the genus *Weissella,* the genus *Propionibacterium,* the genus *Lysinibacillus,* the genus *Stenotrophomonas,* the genus *Arthrobacter,* the genus *Comamonas,* the genus *Marinobacter,* the genus *Clostridium,* the genus *Planctomyces,* the genus *Luteolibacter,* the genus *Delftia,* the genus *Agrobacterium,* the genus *Rhodoplanes,* the genus DA101, the genus *Gemmata,* the genus *Coprobacillus,* the genus *Arcobacter,* the genus *Helicobacter,* the genus *Candidatus Solibacter,* the genus *Methanosarcina,* the genus *Thermacetogenium,* the genus *Synechococcus,* the genus *Desulfomicrobium,* the genus *Chthoniobacter,* the genus *Aminobacterium,* the genus *Gallicola,* the genus *Anaeromyxobacter,* the genus *Muricauda,* and the genus *Candidatus Koribacter* as a biomarker exhibited significant diagnostic performance for Parkinson's disease (see Table 6 and FIG. 6).

**[Table 6]**

| | Control | | Parkinson's disease | | t-test | | | | |
|---|---|---|---|---|---|---|---|---|---|
| Taxon | Mean | SD | Mean | SD | p-value | Ratio | AUC | Sensiti vity | Specifi city |
| g_Collinsella | 0.0050 | 0.0066 | 0.0000 | 0.0000 | 0.0000 | 0.00 | 0.88 | 0.74 | 1.00 |
| g_Adlercreutzia | 0.0013 | 0.0021 | 0.0000 | 0.0000 | 0.0001 | 0.00 | 0.80 | 0.59 | 1.00 |
| g_SMB53 | 0.0026 | 0.0037 | 0.0000 | 0.0000 | 0.0000 | 0.00 | 0.83 | 0.68 | 0.87 |
| g_Proteus | 0.0192 | 0.0298 | 0.0000 | 0.0000 | 0.0000 | 0.00 | 0.97 | 0.91 | 1.00 |
| g_Exiguobacteriu m | 0.0029 | 0.0064 | 0.0000 | 0.0000 | 0.0002 | 0.00 | 0.74 | 0.87 | 0.23 |
| g_Enterococcus | 0.0086 | 0.0099 | 0.0001 | 0.0003 | 0.0000 | 0.02 | 0.93 | 0.84 | 0.92 |
| g_Acinetobacter | 0.0690 | 0.1047 | 0.0020 | 0.0022 | 0.0000 | 0.03 | 0.97 | 0.93 | 0.97 |
| g_Turicibacter | 0.0017 | 0.0025 | 0.0001 | 0.0004 | 0.0000 | 0.04 | 0.75 | 0.99 | 0.08 |
| g_Klebsiella | 0.0009 | 0.0016 | 0.0000 | 0.0002 | 0.0000 | 0.04 | 0.70 | 0.97 | 0.10 |
| g_Lautropia | 0.0015 | 0.0028 | 0.0001 | 0.0003 | 0.0000 | 0.05 | 0.75 | 0.92 | 0.26 |
| g_Akkermansia | 0.0216 | 0.0271 | 0.0012 | 0.0025 | 0.0000 | 0.06 | 0.88 | 0.82 | 0.82 |
| g_Parabacteroide s | 0.0115 | 0.0156 | 0.0007 | 0.0015 | 0.0000 | 0.06 | 0.84 | 0.75 | 0.82 |
| g_Rhizobium | 0.0054 | 0.0061 | 0.0004 | 0.0014 | 0.0000 | 0.07 | 0.93 | 0.84 | 0.92 |
| g _Actinomyces | 0.0059 | 0.0095 | 0.0005 | 0.0008 | 0.0000 | 0.09 | 0.83 | 0.78 | 0.69 |
| g_Lactococcus | 0.0048 | 0.0064 | 0.0005 | 0.0009 | 0.0000 | 0.10 | 0.76 | 0.75 | 0.56 |
| g_Blautia | 0.0049 | 0.0067 | 0.0005 | 0.0009 | 0.0000 | 0.10 | 0.80 | 0.70 | 0.72 |
| g_Veillonella | 0.0096 | 0.0188 | 0.0010 | 0.0014 | 0.0002 | 0.11 | 0.78 | 0.75 | 0.62 |
| g_Pseudomonas | 0.0916 | 0.0826 | 0.0129 | 0.0070 | 0.0000 | 0.14 | 0.98 | 0.95 | 0.92 |
| g_Rothia | 0.0043 | 0.0058 | 0.0007 | 0.0014 | 0.0000 | 0.15 | 0.73 | 0.76 | 0.46 |
| g_Dorea | 0.0023 | 0.0037 | 0.0004 | 0.0011 | 0.0000 | 0.15 | 0.75 | 0.93 | 0.21 |
| g_Streptococcus | 0.0272 | 0.0210 | 0.0044 | 0.0033 | 0.0000 | 0.16 | 0.89 | 0.83 | 0.85 |
| g_Haemophilus | 0.0049 | 0.0059 | 0.0009 | 0.0011 | 0.0000 | 0.18 | 0.75 | 0.71 | 0.56 |
| g_Enhydrobacter | 0.0108 | 0.0104 | 0.0031 | 0.0036 | 0.0000 | 0.28 | 0.78 | 0.84 | 0.56 |
| g_Rhodococcus | 0.0018 | 0.0028 | 0.0006 | 0.0012 | 0.0018 | 0.32 | 0.72 | 0.99 | 0.10 |
| g_Coprococcus | 0.0025 | 0.0026 | 0.0009 | 0.0017 | 0.0002 | 0.37 | 0.75 | 0.97 | 0.15 |
| g_Oscillospira | 0.0045 | 0.0048 | 0.0101 | 0.0055 | 0.0000 | 2.24 | 0.81 | 0.89 | 0.44 |
| g_Ruminococcus | 0.0041 | 0.0042 | 0.0108 | 0.0058 | 0.0000 | 2.62 | 0.83 | 0.89 | 0.51 |
| g_Bacteroides | 0.0405 | 0.0357 | 0.1142 | 0.0288 | 0.0000 | 2.82 | 0.94 | 0.95 | 0.87 |
| g_Corynebacteri um | 0.0103 | 0.0104 | 0.0348 | 0.0154 | 0.0000 | 3.37 | 0.92 | 0.89 | 0.72 |
| g_Weissella | 0.0014 | 0.0020 | 0.0046 | 0.0034 | 0.0000 | 3.37 | 0.85 | 0.91 | 0.62 |
| g_Propionibacter ium | 0.0101 | 0.0087 | 0.0548 | 0.0434 | 0.0000 | 5.43 | 0.98 | 0.96 | 0.92 |
| g_Lysinibacillus | 0.0002 | 0.0011 | 0.0011 | 0.0014 | 0.0008 | 5.46 | 0.78 | 0.95 | 0.46 |
| g_Stenotrophom onas | 0.0005 | 0.0012 | 0.0029 | 0.0029 | 0.0000 | 6.05 | 0.87 | 0.93 | 0.46 |
| g_Arthrobacter | 0.0002 | 0.0011 | 0.0012 | 0.0019 | 0.0028 | 6.37 | 0.73 | 0.97 | 0.28 |
| g_Comamonas | 0.0002 | 0.0007 | 0.0017 | 0.0016 | 0.0000 | 7.76 | 0.83 | 0.95 | 0.51 |
| g_Marinobacter | 0.0001 | 0.0005 | 0.0005 | 0.0008 | 0.0030 | 8.31 | 0.71 | 0.99 | 0.31 |
| g_Clostridium | 0.0006 | 0.0012 | 0.0079 | 0.0055 | 0.0000 | 13.95 | 0.95 | 0.93 | 0.77 |
| g_Planctomyces | 0.0001 | 0.0006 | 0.0018 | 0.0021 | 0.0000 | 18.78 | 0.82 | 0.97 | 0.49 |
| g_Luteolibacter | 0.0001 | 0.0003 | 0.0025 | 0.0025 | 0.0000 | 32.91 | 0.96 | 0.96 | 0.74 |
| g_Delftia | 0.0002 | 0.0011 | 0.0091 | 0.0063 | 0.0000 | 39.85 | 0.99 | 0.97 | 0.90 |
| g_Agrobacterium | 0.0005 | 0.0011 | 0.0226 | 0.0108 | 0.0000 | 45.62 | 1.00 | 0.99 | 0.95 |
| g_Rhodoplanes | 0.0000 | 0.0001 | 0.0036 | 0.0040 | 0.0000 | 120.50 | 0.86 | 0.99 | 0.62 |
| g_DA101 | 0.0000 | 0.0002 | 0.0039 | 0.0028 | 0.0000 | 153.39 | 0.97 | 0.99 | 0.79 |
| g_Gemmata | 0.0000 | 0.0001 | 0.0019 | 0.0028 | 0.0002 | 157.09 | 0.80 | 0.99 | 0.46 |
| g_Coprobacillus | 0.0000 | 0.0000 | 0.0014 | 0.0015 | 0.0000 | 246.64 | 0.88 | 0.99 | 0.62 |
| g_Arcobacter | 0.0000 | 0.0000 | 0.0008 | 0.0015 | 0.0031 | 335.36 | 0.75 | 0.99 | 0.33 |
| g_Helicobacter | 0.0000 | 0.0000 | 0.0006 | 0.0013 | 0.0072 | 770.90 | 0.70 | 0.99 | 0.28 |
| g_Candidatus Solibacter | 0.0000 | 0.0000 | 0.0022 | 0.0024 | 0.0000 | 12365.5 8 | 0.89 | 0.99 | 0.77 |
| g_Methanosarcin a | 0.0000 | 0.0000 | 0.0014 | 0.0022 | 0.0000 | | 0.98 | 1.00 | 0.90 |
| g_Thermacetoge nium | 0.0000 | 0.0000 | 0.0050 | 0.0028 | 0.0000 | | 0.93 | 0.91 | 0.95 |
| g_Synechococcu s | 0.0000 | 0.0000 | 0.0021 | 0.0027 | 0.0000 | | 0.91 | 1.00 | 0.79 |
| g_Desulfomicrob ium | 0.0000 | 0.0000 | 0.0011 | 0.0014 | 0.0000 | | 0.88 | 1.00 | 0.69 |
| g_Chthoniobacte | 0.0000 | 0.0000 | 0.0008 | 0.0012 | 0.0000 | | 0.88 | 1.00 | 0.69 |
| r | | | | | | | | | |
| g_Aminobacteriu m | 0.0000 | 0.0000 | 0.0008 | 0.0012 | 0.0000 | | 0.87 | 1.00 | 0.69 |
| g_Gallicola | 0.0000 | 0.0000 | 0.0007 | 0.0012 | 0.0000 | | 0.76 | 1.00 | 0.44 |
| g_Anaeromyxob acter | 0.0000 | 0.0000 | 0.0006 | 0. 0011 | 0.0000 | | 0.75 | 1.00 | 0.44 |
| g_Muricauda | 0.0000 | 0.0000 | 0.0007 | 0.0014 | 0.0000 | | 0.74 | 1.00 | 0.46 |
| g_Candidatus Koribacter | 0.0000 | 0.0000 | 0.0005 | 0.0011 | 0.0001 | | 0.72 | 1.00 | 0.41 |

### [industrial Applicability]

A method of diagnosing Parkinson's disease diagnosis through bacterial metagenomic analysis, according to the present invention, can be used to diagnose Parkinson's disease by analyzing an increase or decrease in content of extracellular vesicles derived from specific bacteria through bacterial metagenomic analysis using a subject-derived urine sample. Extracellular vesicles secreted from microorganisms such as bacteria, archaea, and the like present in the environment are absorbed into the human body and distributed in the brain, and thus can directly affect inflammatory responses and brain functions, and since it is difficult to implement early diagnosis for Parkinson's disease, which is characterized by inflammation, before symptoms appear, efficient treatment is difficult. Thus, according to the present invention Parkinson's disease may be diagnosed through metagenomic analysis of bacteria-derived extracellular vesicles using a human body-derived urine sample, and thus the onset of Parkinson's disease can be delayed or prevented through appropriate management by early diagnosis, and, even after Parkinson's disease occurs, early diagnosis for Parkinson's disease can be implemented, thereby lowering the incidence rate of Parkinson's disease and increasing therapeutic effects. In addition, patients diagnosed with Parkinson's disease are able to avoid exposure to causative factors predicted through bacterial metagenomic analysis according to the present invention, whereby the progression of Parkinson's disease is ameliorated, or recurrence thereof can be prevented.

## Claims

1. A method of diagnosing Parkinson's disease, the method comprising:
(a) extracting DNA from extracellular vesicles isolated from a subject sample;
(b) performing polymerase chain reaction (PCR) on the extracted DNA using a pair of primers having SEQ ID NO:1 and SEQ ID NO: 2; and
(c) comparing an increase or decrease in content of bacteria-derived extracellular vesicles of the subject sample with that of a normal individual-derived sample through sequencing of a product of the PCR, wherein said extracellular vesicles are derived from:
one or more bacteria selected from the group consisting of the phylum *Proteobacteria,* the phylum *Cyanobacteria,* the phylum *Gemmatimonadetes,* the phylum *Chloroflexi,* the phylum *Synergistetes,* the phylum *Acidobacteria,* the phylum *Planctomycetes,* the phylum *Parvarchaeota,* the phylum *Chlorobi,* and the phylum *Thermotogae;*
one or more bacteria selected from the group consisting of the class *Coriobacteriia,* the class *Gammaproteobacteria,* the class *Verrucomicrobiae,* the class *Actinobacteria,* the class *Alphaproteobacteria,* the class *Chloroplast,* the class *Saprospirae,* the class *Deltaproteobacteria,* the class *Epsilonproteobacteria,* the class Ellin6529, the class *Chloracidobacteria,* the class *Opitutae,* the class *Thermoleophilia,* the class *Synergistia,* the class *Gemmatimonadetes,* the class *Planctomycetia,* the class *Acidobacteriia,* the class *Solibacteres,* the class *Anaerolineae,* the class *Chloroflexi,* the class *Phycisphaerae,* the class *Synechococcophycideae,* the class *Acidimicrobiia,* the class *Acidobacteria*-6, the class *Spartobacteria,* the class *Pedosphaerae,* the class *Ktedonobacteria,* and the class *Dehalococcoidetes;*
one or more bacteria selected from the group consisting of the order *Turicibacterales,* the order *Pseudomonadales,* the order *Coriobacteriales,* the order *Pasteurellales,* the order *Enterobacteriales,* the order *Verrucomicrobiales,* the order *Gemellales,* the order *Neisseriales,* the order *Saprospirales,* the order *Actinomycetales,* the order *Streptophyta,* the order *Rhizobiales,* the order *Rhodospirillales,* the order *Xanthomonadales,* the order *Myxococcales,* the order *Campylobacterales,* the order *Desulfovibrionales,* the order *Solirubrobacterales,* the order *Opitutales,* the order *Rickettsiales,* the order *Pirellulales,* the order *Synergistales,* the order *Planctomycetales,* the order *Acidobacteriales,* the order *Solibacterales,* the order *Gaiellales,* the order *Gemmatales,* the order *Acidimicrobiales,* the order *Chthoniobacterales,* the order *Thermoanaerobacterales,* the order *Pedosphaerales,* the order *Phycisphaerales,* the order Sediment-1, the order *Chlorophyta,* the order *Synechococcales,* the order *Roseiflexales,* the order Ellin329, the order *Anaerolineales,* the order Ellin5290, the order *Cryptophyta,* and the order *Thermotogales;*
one or more bacteria selected from the group consisting of the family *Exiguobacteraceae,* the family *Enterococcaceae,* the family *Turicibacteraceae,* the family *Mogibacteriaceae,* the family *Moraxellaceae,* the family *Porphyromonadaceae,* the family *Burkholderiaceae,* the family *Actinomycetaceae,* the family *Coriobacteriaceae,* the family *Methylobacteriaceae,* the family *Streptococcaceae,* the family *Pseudomonadaceae,* the family *Pasteurellaceae,* the family *Veillonellaceae,* the family *Peptostreptococcaceae,* the family *Enterobacteriaceae,* the family *Verrucomicrobiaceae,* the family *Lachnospiraceae,* the family *Bradyrhizobiaceae,* the family *Rikenellaceae,* the family *Tissierellaceae,* the family *Bacteroidaceae,* the family *Chitinophagaceae,* the family *Corynebacteriaceae,* the family *Xanthomonadaceae,* the family *Rhizobiaceae,* the family *Propionibacteriaceae,* the family *Desulfobacteraceae,* the family *Barnesiellaceae,* the family *Comamonadaceae,* the family *mitochondria,* the family *Hyphomicrobiaceae,* the family *Alteromonadaceae,* the family *Sinobacteraceae,* the family *Pirellulaceae,* the family *Dethiosulfovibrionaceae,* the family *Acidobacteriaceae,* the family *Planctomycetaceae,* the family *Isosphaeraceae,* the family *Gaiellaceae,* the family *Koribacteraceae,* the family *Helicobacteraceae,* the family *Chthoniobacteraceae,* the family *Gemmataceae,* the family *Solibacteraceae,* the family *Pelagibacteraceae,* the family Ellin515, the family *Thermoanaerobacteraceae,* the family *Methanoregulaceae,* the family *Synechococcaceae,* the family *Desulfomicrobiaceae,* the family *Kouleothrixaceae,* the family *Alicyclobacillaceae,* the family *Myxococcaceae,* the family *Anaerolinaceae,* and the family *Desulfohalobiaceae*; or
one or more bacteria selected from the group consisting of the genus *Collinsella,* the genus *Adlercreutzia,* the genus *Exiguobacterium,* the genus *Enterococcus,* the genus *Acinetobacter,* the genus *Turicibacter,* the genus *Klebsiella,* the genus *Lautropia,* the genus *Akkermansia,* the genus *Parabacteroides,* the genus *Rhizobium,* the genus *Actinomyces,* the genus *Veillonella,* the genus *Pseudomonas,* the genus *Rothia,* the genus *Dorea,* the genus *Streptococcus,* the genus *Haemophilus,* the genus *Enhydrobacter,* the genus *Rhodococcus,* the genus *Coprococcus,* the genus *Oscillospira,* the genus *Ruminococcus,* the genus *Bacteroides,* the genus *Corynebacterium,* the genus *Weissella,* the genus *Propionibacterium,* the genus *Lysinibacillus,* the genus *Stenotrophomonas,* the genus *Arthrobacter,* the genus *Comamonas,* the genus *Marinobacter,* the genus *Clostridium,* the genus *Planctomyces,* the genus *Luteolibacter,* the genus *Delftia,* the genus *Agrobacterium,* the genus *Rhodoplanes,* the genus *Gemmata,* the genus *Coprobacillus,* the genus *Arcobacter,* the genus *Helicobacter,* the genus *Candidatus Solibacter,* the genus *Methanosarcina,* the genus *Thermacetogenium,* the genus *Synechococcus,* the genus *Desulfomicrobium,* the genus *Chthoniobacter,* the genus *Aminobacterium,* the genus *Gallicola,* the genus *Anaeromyxobacter,* the genus *Muricauda,* and the genus *Candidatus Koribacter,*
wherein the subject sample is urine.

2. The method of claim 1, wherein said extracellular vesicles are derived from one or more bacteria selected from the group consisting of the genus *Collinsella,* the genus *Adlercreutzia,* the genus *Exiguobacterium,* the genus *Enterococcus,* the genus *Acinetobacter,* the genus *Turicibacter,* the genus *Lautropia,* the genus *Akkermansia,* the genus *Parabacteroides,* the genus *Rhizobium,* the genus *Actinomyces,* the genus *Veillonella,* the genus *Pseudomonas,* the genus *Rothia,* the genus *Dorea,* the genus *Streptococcus,* the genus *Haemophilus,* the genus *Enhydrobacter,* the genus *Rhodococcus,* the genus *Coprococcus,* the genus *Oscillospira,* the genus *Ruminococcus,* the genus *Bacteroides,* the genus *Corynebacterium,* the genus *Weissella,* the genus *Propionibacterium,* the genus *Lysinibacillus,* the genus *Stenotrophomonas,* the genus *Arthrobacter,* the genus *Comamonas,* the genus *Clostridium,* the genus *Planctomyces,* the genus *Luteolibacter,* the genus *Delftia,* the genus *Agrobacterium,* the genus *Rhodoplanes,* the genus *Gemmata,* the genus *Coprobacillus,* the genus *Candidatus Solibacter,* the genus *Methanosarcina,* the genus *Thermacetogenium,* the genus *Synechococcus,* and the genus *Desulfomicrobium.*

## Patentansprüche

1. Verfahren zur Diagnose der Parkinson-Krankheit, wobei das Verfahren Folgendes umfasst:
(a) Extrahieren von DNA aus extrazellulären Vesikeln, die aus einer Patientenprobe isoliert wurden;
(b) Durchführen einer Polymerase-Kettenreaktion (PCR) an der extrahierten DNA unter Verwendung eines Paars von Primern, die SEQ ID Nr: 1 und SEQ ID Nr: 2 aufweisen; und
(c) Vergleichen eines Anstiegs oder einer Abnahme des Gehalts an von Bakterien stammenden extrazellulären Vesikeln der Patientenprobe mit dem einer normalen, von einer Person stammenden Probe durch Sequenzierung eines Produkts der PCR, wobei die extrazellulären Vesikel stammen von:
einem oder mehreren Bakterien, ausgewählt aus der Gruppe bestehend aus dem Stamm *Proteobacteria,* dem Stamm *Cyanobacteria,* dem Stamm *Gemmatimonadetes,* dem Stamm *Chloroflexi,* dem Stamm *Synergistetes,* dem Stamm *Acidobacleria,* dem Stamm *Planctomycetes,* dem Stamm *Parvarchaeota,* dem Stamm *Chlorobi,* und dem Stamm *Thermotogae;*
einem oder mehreren Bakterien, ausgewählt aus der Gruppe bestehend aus der Klasse *Coriobacteriia,* der Klasse *Gammaproteobacteria,* der Klasse *Verrucomicrobiae,* der Klasse *Actinobacteria,* der Klasse *Alphaproteobacteria,* der Klasse *Chloroplast,* der Klasse *Saprospirae,* der Klasse *Deltaproteobacteria,* der Klasse *Epsilonproteobacteria,* der Klasse *Ellin6529,* der Klasse *Chloracidobacteria,* der Klasse *Opitutae,* der Klasse *Thermoleophilia,* der Klasse *Synergistia,* der Klasse *Gemmatimonadetes,* der Klasse *Planctomycetia,* der Klasse *Acidobacteriia,* der Klasse *Solibacteres,* der Klasse *Anaerolineae,* der Klasse *Chloroflexi,* der Klasse *Phycisphaerae,* der Klasse *Synechococcophycideae,* der Klasse *Acidimicrobiia,* der Klasse *Acidobacleria-6,* der Klasse *Spartobacteria,* der Klasse *Pedosphaerae,* der Klasse *Ktedonobacteria,* und der Klasse *Dehalococcoidetes*;
einem oder mehreren Bakterien, ausgewählt aus der Gruppe bestehend aus der Ordnung *Turicibacterales,* der Ordnung *Pseudomonadales,* der Ordnung *Coriobacteriales,* der Ordnung *Pasteurellales,* der Ordnung *Enterobacteriales,* der Ordnung *Verrucomicrobiales,* der Ordnung *Gemellales,* der Ordnung *Neisseriales,* der Ordnung *Saprospirales,* der Ordnung *Actinomycetales,* der Ordnung *Streptophyta,* der Ordnung *Rhizobiales,* der Ordnung *Rhodospirillales,* der Ordnung *Xanthomonadales,* der Ordnung *Myxococcales,* der Ordnung *Campylobacter ales,* der Ordnung *Desulfovibrionales,* der Ordnung *Solirubrobacterales,* der Ordnung *Opitutales,* der Ordnung *Rickettsiales,* der Ordnung *Pirellulales,* der Ordnung *Synergistales,* der Ordnung *Planctomycetales,* der Ordnung *Acidobacteriales,* der Ordnung *Solibacterales,* der Ordnung *Gaiellales,* der Ordnung *Gemmatales,* der Ordnung *Acidimicrobiales,* der Ordnung *Chthoniobacterales,* der Ordnung *Thermoanaerobacterales,* der Ordnung *Pedosphaerales,* der Ordnung *Phycisphaerales,* der Ordnung *Sediment-1,* der Ordnung *Chlorophyta,* der Ordnung *Synechococcales,* der Ordnung *Roseiflexales,* der Ordnung *Ellin329,* der Ordnung *Anaerolineales,* der Ordnung *Ellin5290,* der Ordnung *Cryptophyta,* und der Ordnung *Thermotogales*;
einem oder mehreren Bakterien, ausgewählt aus der Gruppe bestehend aus der Familie *Exiguobacteraceae,* der Familie *Enterococcaceae,* der Familie *Turicibacteraceae,* der Familie *Mogibacteriaceae,* der Familie *Moraxellaceae,* der Familie *Porphyromonadaceae,* der Familie *Burkholderiaceae,* der Familie *Actinomycetaceae,* der Familie *Coriobacteriaceae,* der Familie *Methylobacteriaceae,* der Familie *Streptococcaceae,* der Familie *Pseudomonadaceae,* der Familie *Pasteurellaceae,* der Familie *Veillonellaceae,* der Familie *Peptostreptococcaceae,* der Familie *Enterobacteriaceae,* der Familie *Verrucomicrobiaceae,* der Familie *Lachnospiraceae,* der Familie *Bradyrhizobiaceae,* der Familie *Rikenellaceae,* der Familie *Tissierellaceae,* der Familie *Bacteroidaceae,* der Familie *Chitinophagaceae,* der Familie *Corynebacteriaceae,* der Familie *Xanthomonadaceae,* der Familie *Rhizobiaceae,* der Familie *Propionibacteriaceae,* der Familie *Desulfobacteraceae,* der Familie *Barnesiellaceae,* der Familie *Comamonadaceae,* der Familie *Mitochondria,* der Familie *Hyphomicrobiaceae,* der Familie *Alteromonadaceae,* der Familie *Sinobacleraceae,* der Familie *Pirellulaceae,* der Familie *Dethiosulfovibrionaceae,* der Familie *Acidobacleriaceae,* der Familie *Planctomycetaceae,* der Familie *Isosphaeraceae,* der Familie *Gaiellaceae,* der Familie *Koribacleraceae,* der Familie *Helicobacteraceae,* der Familie *Chthoniobacteraceae,* der Familie *Gemmataceae,* der Familie *Solibacteraceae,* der Familie *Pelagibacteraceae,* der Familie *Ellin515,* der Familie *Thermoanaerobacleraceae,* der Familie *Methanoregulaceae,* der Familie *Synechococcaceae,* der Familie *Desulfomicrobiaceae,* der Familie *Kouleothrixaceae,* der Familie *Alicyclobacillaceae,* der Familie *Myxococcaceae,* der Familie *Anaerolinaceae,* und der Familie *Desulfohalobiaceae:* oder
einem oder mehreren Bakterien, ausgewählt aus der Gruppe bestehend aus der Gattung *Collinsella,* der Gattung *Adlercreutzia,* der Gattung *Exiguobacterium,* der Gattung *Enterococcus,* der Gattung *Acinetobacter,* der Gattung *Turicibacter,* der Gattung *Klebsiella,* der Gattung *Lautropia,* der Gattung *Akkermansia,* der Gattung *Parabacteroides,* der Gattung *Rhizobium,* der Gattung *Actinomyces,* der Gattung *Veillonella,* der Gattung *Pseudomonas,* der Gattung *Rothia,* der Gattung *Dorea,* der Gattung *Streptococcus,* der Gattung *Haemophilus,* der Gattung *Enhydrobacter,* der Gattung *Rhodococcus,* der Gattung *Coprococcus,* der Gattung *Oscillospira,* der Gattung *Ruminococcus,* der Gattung *Bacteroides,* der Gattung *Corynebacterium,* der Gattung *Weissella,* der Gattung *Propionibacterium,* der Gattung *Lysinibacillus,* der Gattung *Stenotrophomonas,* der Gattung *Arthrobacter,* der Gattung *Comamonas,* der Gattung *Marinobacter,* der Gattung *Clostridium,* der Gattung *Planctomyces,* der Gattung *Luteolibacter,* der Gattung *Delftia,* der Gattung *Agrobacterium,* der Gattung *Rhodoplanes,* der Gattung *Gemmata,* der Gattung *Coprobacillus,* der Gattung *Arcobacter,* der Gattung *Helicobacter,* der Gattung *Candidatus Solibacter,* der Gattung *Methanosarcina,* der Gattung *Thermacetogenium,* der Gattung *Synechococcus,* der Gattung *Desulfomicrobium,* der Gattung *Chthoniobacter,* der Gattung *Aminobacterium,* der Gattung *Gallicola,* der Gattung *Anaeromyxobacter,* der Gattung *Muricauda,* und der Gattung *Candidatus Koribacter,*
wobei die Patientenprobe Urin ist.

2. Verfahren nach Anspruch 1, wobei die extrazellulären Vesikel von einem oder mehreren Bakterien stammen, die ausgewählt sind aus der Gruppe bestehend aus der Gattung *Collinsella,* der Gattung *Adlercreutzia,* der Gattung *Exiguobacterium,* der Gattung *Enterococcus,* der Gattung *Acinetobacter,* der Gattung *Turicibacter,* der Gattung *Lautropia,* der Gattung *Akkermansia,* der Gattung *Parabacteroides,* der Gattung *Rhizobium,* der Gattung *Actinomyces,* der Gattung *Veillonella,* der Gattung *Pseudomonas,* der Gattung *Rothia,* der Gattung *Dorea,* der Gattung *Streptococcus,* der Gattung *Haemophilus,* der Gattung *Enhydrobacter,* der Gattung *Rhodococcus,* der Gattung *Coprococcus,* der Gattung *Oscillospira,* der Gattung *Ruminococcus,* der Gattung *Bacteroides,* der Gattung *Corynebacterium,* der Gattung *Weissella,* der Gattung *Propionibacterium,* der Gattung *Lysinibacillus,* der Gattung *Stenotrophomonas,* der Gattung *Arthrobacter,* der Gattung *Comamonas,* der Gattung *Clostridium,* der Gattung *Planctomyces,* der Gattung *Luteolibacter,* der Gattung *Delftia,* der Gattung *Agrobacterium,* der Gattung *Rhodoplanes,* der Gattung *Gemmata,* der Gattung *Coprobacillus,* der Gattung *Candidatus Solibacter,* der Gattung *Methanosarcina,* der Gattung *Thermacetogenium,* der Gattung *Synechococcus,* und der Gattung *Desulfomicrobium.*

## Revendications

1. Procédé de diagnostic de la maladie de Parkinson, le procédé comprenant :
(a) une extraction d'ADN de vésicules extracellulaires isolées à partir d'un échantillon de sujet ;
(b) une réalisation d'une amplification en chaîne par polymérase (PCR) sur l'ADN extrait en utilisant une paire d'amorces présentant la SEQ ID NO : 1 et la SEQ ID NO : 2 ; et
(c) une comparaison d'une augmentation ou diminution de la teneur en vésicules extracellulaires dérivées de bactéries de l'échantillon de sujet avec celle d'un échantillon dérivé d'un individu normal par l'intermédiaire d'un séquençage d'un produit de la PCR, dans lequel lesdites vésicules extracellulaires sont dérivées de :
une ou plusieurs bactéries sélectionnées dans le groupe consistant en phylum Proteobacteria, phylum Cyanobacteria, phylum Gemmatimonadetes, phylum Chloroflexi, phylum Synergistetes, phylum Acidobacteria, phylum Planctomycetes, phylum Parvarchaeota, phylum Chlorobi et phylum Thermotogae ;
une ou plusieurs bactéries sélectionnées dans le groupe consistant en classe Coriobacteriia, classe Gammaproteobacteria, classe Verrucomicrobiae, classe Actinobacteria, classe Alphaproteobacteria, classe Chloroplast, classe Saprospirae, classe Deltaproteobacteria, classe Epsilonproteobacteria, classe Ellin6529, classe Chloracidobacteria, classe Opitutae, classe Thermoleophilia, classe Synergistia, classe Gemmatimonadetes, classe Planctomycetia, classe Acidobacteriia, classe Solibacteres, classe Anaerolineae, classe Chloroflexi, classe Phycisphaerae, classe Synechococcophycideae, classe Acidimicrobiia, classe Acidobacteria-6, classe Spartobacteria, classe Pedosphaerae, classe Ktedonobacteria et classe Dehalococcoidetes ;
une ou plusieurs bactéries sélectionnées dans le groupe consistant en ordre Turicibacterales, ordre Pseudomonadales, ordre Coriobacteriales, ordre Pasteurellales, ordre Enterobacteriales, ordre Verrucomicrobiales, ordre Gemellales, ordre Neisseriales, ordre Saprospirales, ordre Actinomycetales, ordre Streptophyta, ordre Rhizobiales, ordre Rhodospirillales, ordre Xanthomonadales, ordre Myxococcales, ordre Campylobacterales, ordre Desulfovibrionales, ordre Solirubrobacterales, ordre Opitutales, ordre Rickettsiales, ordre Pirellulales, ordre Synergistales, ordre Planctomycetales, ordre Acidobacteriales, ordre Solibacterales, ordre Gaiellales, ordre Gemmatales, ordre Acidimicrobiales, ordre Chthoniobacterales, ordre Thermoanaerobacterales, ordre Pedosphaerales, ordre Phycisphaerales, ordre Sediment-1, ordre Chlorophyta, ordre Synechococcales, ordre Roseiflexales, ordre Ellin329, ordre Anaerolineales, ordre Ellin5290, ordre Cryptophyta et ordre Thermotogales ;
une ou plusieurs bactéries sélectionnées dans le groupe consistant en famille Exiguobacteraceae, famille Enterococcaceae, famille Turicibacteraceae, famille Mogibacteriaceae, famille Moraxellaceae, famille Porphyromonadaceae, famille Burkholderiaceae, famille Actinomycetaceae, famille Coriobacteriaceae, famille Methylobacteriaceae, famille Streptococcaceae, famille Pseudomonadaceae, famille Pasteurellaceae, famille Veillonellaceae, famille Peptostreptococcaceae, famille Enterobacteriaceae, famille Verrucomicrobiaceae, famille Lachnospiraceae, famille Bradyrhizobiaceae, famille Rikenellaceae, famille Tissierellaceae, famille Bacteroidaceae, famille Chitinophagaceae, famille Corynebacteriaceae, famille Xanthomonadaceae, famille Rhizobiaceae, famille Propionibacteriaceae, famille Desulfobacteraceae, famille Barnesiellaceae, famille Comamonadaceae, famille mitochondria, famille Hyphomicrobiaceae, famille Alteromonadaceae, famille Sinobacteraceae, famille Pirellulaceae, famille Dethiosulfovibrionaceae, famille Acidobacteriaceae, famille Planctomycetaceae, famille Isosphaeraceae, famille Gaiellaceae, famille Koribacteraceae, famille Helicobacteraceae, famille Chthoniobacteraceae, famille Gemmataceae, famille Solibacteraceae, famille Pelagibacteraceae, famille Ellin515, famille Thermoanaerobacteraceae, famille Methanoregulaceae, famille Synechococcaceae, famille Desulfomicrobiaceae, famille Kouleothrixaceae, famille Alicyclobacillaceae, famille Myxococcaceae, famille Anaerolinaceae et famille Desulfohalobiaceae ; ou
une ou plusieurs bactéries sélectionnées dans le groupe consistant en genre Collinsella, genre Adlercreutzia, genre Exiguobacterium, genre Enterococcus, genre Acinetobacter, genre Turicibacter, genre Klebsiella, genre Lautropia, genre Akkermansia, genre Parabacteroides, genre Rhizobium, genre Actinomyces, genre Veillonella, genre Pseudomonas, genre Rothia, genre Dorea, genre Streptococcus, genre Haemophilus, genre Enhydrobacter, genre Rhodococcus, genre Coprococcus, genre Oscillospira, genre Ruminococcus, genre Bacteroides, genre Corynebacterium, genre Weissella, genre Propionibacterium, genre Lysinibacillus, genre Stenotrophomonas, genre Arthrobacter, genre Comamonas, genre Marinobacter, genre Clostridium, genre Planctomyces, genre Luteolibacter, genre Delftia, genre Agrobacterium, genre Rhodoplanes, genre Gemmata, genre Coprobacillus, genre Arcobacter, genre Helicobacter, genre Candidatus Solibacter, genre Methanosarcina, genre Thermacetogenium, genre Synechococcus, genre Desulfomicrobium, genre Chthoniobacter, genre Aminobacterium, genre Gallicola, genre Anaeromyxobacter, genre Muricauda et genre Candidatus Koribacter,
dans lequel l'échantillon de sujet est de l'urine.

2. Procédé selon la revendication 1, dans lequel lesdites vésicules extracellulaires sont dérivées d'une ou plusieurs bactéries sélectionnées dans le groupe consistant en genre Collinsella, genre Adlercreutzia, genre Exiguobacterium, genre Enterococcus, genre Acinetobacter, genre Turicibacter, genre Lautropia, genre Akkermansia, genre Parabacteroides, genre Rhizobium, genre Actinomyces, genre Veillonella, genre Pseudomonas, genre Rothia, genre Dorea, genre Streptococcus, genre Haemophilus, genre Enhydrobacter, genre Rhodococcus, genre Coprococcus, genre Oscillospira, genre Ruminococcus, genre Bacteroides, genre Corynebacterium, genre Weissella, genre Propionibacterium, genre Lysinibacillus, genre Stenotrophomonas, genre Arthrobacter, genre Comamonas, genre Clostridium, genre Planctomyces, genre Luteolibacter, genre Delftia, genre Agrobacterium, genre Rhodoplanes, genre Gemmata, genre Coprobacillus, genre Candidatus Solibacter, genre Methanosarcina, genre Thermacetogenium, genre Synechococcus et genre Desulfomicrobium.
